# EUROPEAN PATENT APPLICATION

(11) **EP 2 899 280 A1**
(43) Date of publication of application: **29.07.2015**
(21) Application number: 14152848.9
(22) Date of filing: 28.01.2014
(51) Int. Cl.: C12P 7/62, C12N 9/20, C12N 9/04, C12N 9/02

(54) **Process for the enzymatic production of oligo-/polyesters**

(71) Applicant: Ernst-Moritz-Arndt-Universität Greifswald, 17487 Greifswald (DE); Enzymicals AG, 17489 Greifswald (DE)
(72) Inventor: Bornscheuer, Uwe T., 17489 Greifswald (DE); Menyes, Ulf, 17509 Neu Boltenhagen (DE); Schmidt, Sandy, 17489 Greifswald (DE); Wardenga, Rainer, 17489 Greifswald (DE); Borchert, Sonja, 17489 Greifswald (DE)
(74) Representative: Wablat Lange Karthaus

(57) **Abstract**

The present invention is related to a method for producing polyesters, wherein a lactone is converted to a polyester by a lipase, characterized
- in that the lipase is Lipase A from *Candida antarctica* (CAL-A) according to SEQ ID NO 1 or a homologue enzyme having sequence identity of at least 25% with SEQ ID NO 1 and having the same function as CAL-A and
- in that the conversion is done in an essentially aqueous medium.

## Description

The present invention is related to a method for producing polyesters, wherein a lactone is converted to a oligo-/polyester by a lipase, using a Lipase A from *Candida antarctica* or a homologue enzyme wherein the conversion is done in an essentially aqueous medium.

Polyesters such as polycaprolactone (PCL) are important polymers due to their mechanical, thermal and physical properties and thus allow versatile applications. In contrast to many other synthetic polymers, polycaprolactone shows several special properties like its biodegradability (Gross & Kalra, 2002), semicrystallinity (Iroh, 1999), the rare property of being miscible with many other polymers like polyvinylchloride or polystyrene-acrylonitrile and also the mechanical compatibility with polyethylene, polypropylene or natural rubber (Labet & Thielemans, 2009).

The biodegradability of PCL depends on the molecular weight, the degree of crystallinity and the environment of degradation (Chandra & Rustgi, 1998; Chen et al, 2000; De Kesel, 1997; Gross & Kalra, 2002; Ikada & Tsuji, 2000; Joshi & Madras, 2008; Lam et al, 2007; Pena et al, 2006; Sinha et al, 2004a). Many microbes are known, which can biodegrade PCL completely (Gross & Kalra, 2002). In contrast to the enzymatically degradation of PCL by microbes, humans are not able to degrade PCL enzymatically in the body (Ikada & Tsuji, 2000). This property makes PCL useful in medical applications e.g. in tissue engineering (Hutmacher et al, 2001; Jenkins et al, 2006; Lam et al, 2007; Pena et al, 2006).

Other fields of applications are long-term drug delivery systems (Chandra & Rustgi, 1998; Chen et al, 2000; Sinha et al, 2004b), microelectronics (Hedrick et al, 2002), as adhesives (Joshi & Madras, 2008) and in packaging (Ikada & Tsuji, 2000).

This wide applicability and the special properties like the controlled degradability, miscibility with other polymers, biocompatibility and potential to be made from monomers de-rived from renewable sources make PCL a very useful polymer (Labet & Thielemans, 2009).

In principle, two different methods can be used for the synthesis of PCL: chemically and enzymatically. The monomer, ε-CL can be produced industrially from the oxidation of cyclohexanone by peracetic acid (Rocca et al, 2003). The preparation of PCL starting from 6-hydroxycaproic acid by polycondensation is described only in a few papers, e.g. by Braud *et a*/*.* (Braud et al, 1998) and leads to a polymer with a relatively low molecular weight and higher polydispersity. In contrast, the preparation of PCL by ring-opening polymerization (ROP) leads to a polymer of higher molecular mass and lower polydispersity. ROP represents a transesterification and is the preferred route for PCL synthesis.

Dependent on the catalyst, four main mechanisms for ROP of lactones have been described: anionic, cationic, monomer-activated or coordination-insertion ROP. The catalytic systems include metals, organic acids or enzymes.

An example for the ROP by an alkali-based catalyst was reported by Bhaw-Luximon *et a*/*.* (Goury et al, 2005). The polymerization of ε-CL was carried out in dioxane using lithium diisopropylamide. They were able to obtain a polymer of medium molecular mass (Mₙ = 5700 g/mol) with completion of polymerization after only a few minutes at 25°C. To address a catalytic system for ROP using organic compounds, a good example are organic acids with a pKₐ value between 3 and 5. Such acids like tartaric acid or citric acid are able to catalyse the ROP of ε-CL in the presence of benzyl alcohol to initiate the reaction (Casas et al, 2004; Persson et al, 2004). The obtained polymer is of average weight of 2800 g/mol.

The enzymatic ring-opening polymerization catalyzed by lipases in organic solvents and its mechanisms have been described (Kobayashi, 1999; Kobayashi et al, 2001; Nobes et al, 1996; MacDonald, 1995). The most popular enzymes for ROP of ε-CL are lipases. Kobayashi *et a*/*.* (Kobayashi, 1999; Kobayashi et al, 1997; Kobayashi et al, 1998a; Kobayashi et al, 1998b; Kobayashi et al, 2001; MacDonald, 1995; Uyama et al, 1995) described in detail the enzyme-catalyzed ROP of lactones and especially of ε-CL using lipases. A wide number of different enzymes from different origins were investigated including *Candida antarctica* lipase B (CAL-B), porcine pancreatic lipase (PPL), lipase from *Pseudomonas aeruginosa* (PA), lipases from *Pseudomonas fluorescens* (lipases Amano P and PF) and a lipase from *Aspergillus niger* (lipase Amano A). Solvents investigated include heptane, benzene, *tert-*butyl methyl ether, 1,4-dioxane, acetonitrile or isooctane. All reactions take place in bulk organic solvents with only a very small aqueous phase (typically far below 5%), as a minimum amount is important to maintain the lipase in an active form. The water content must be carefully controlled as all lipases described above will otherwise catalyze hydrolysis of the lactone instead of the desired polymerization. CAL-B (usually used in immobilized form) was found best for the polymerization of ε-CL in organic solvents. In less than 24 h high conversions could be obtained while more than 10 days were usually needed with other lipases.

Lactones have already been used in lipase-catalyzed polymerizations, e.g. β-propiolactone (Matsumura et al, 1996), β-butyrolactone (Nobes et al, 1996), cyclic diester-D,L-lactide (Matsumura et al, 1997), propyl malolactonate (Panova et al, 2003), γ-butyrolactone (Nobes et al, 1996). The latter lactone cannot be polymerized chemically with aluminoxane as initiator. Also 1,4-dioxane-2-one and δ-valerolactone may serve as starting materials. Larger lactones (macrolides) include 8-octanolide (Kobayashi et al, 1998c), 11-undodecanolide, 12-dodecanolide, 13-tetradodecanolide, w-pentadecalactone or 16-hexadecanolide. Macrocyclic lactones are ingredients in the flavor and fragrance industry.

Although the ring-opening polymerization of lactones in an aqueous system was already described by Inprakhon et al. (2014), Panlawan et al. (2013) and Namekawa et al. (1998), the lipases used (e.g. lipase CAL-B, Novozymes or lipase PS, Amano) resulted in the synthesis of short oligomers of e.g. ε-caprolactone or ω-pentadecalactone. The authors observed that both, direct hydrolysis of the lactone to the corresponding ω-hydroxyalkanoic acid as well as degradation of the oligomer to the dimer or monomer took place. Extended reaction times up to 72 h resulted in complete hydrolysis (see Inprakhon et al. (2014)). This is not surprising as the lipases used are well known to perform hydrolysis preferentially over a synthesis reaction in an aqueous system.

Thus, there is still a need to find new ways to produce the desired polyesters from variable monomers and to simplify these methods, for example, avoid organic solvents, avoid hydrolysis and/or degradation etc..

The object of the invention was the provision of a new method for the production of oligo-/polyesters, which overcomes the drawbacks as mentioned above.

The problem was solved by a method as disclosed in claims 1, 3 and 5. Preferred embodiments are disclosed in the dependant claims. In other words, the problem was solved by a method for producing oligo-/polyesters, wherein a lactone is converted to a oligo-/polyester by a lipase, characterized
- in that the lipase is Lipase A from *Candida antarctica* (CAL-A) according to SEQ ID NO 1 or a homologue enzyme having sequence identity of at least 25% with SEQ ID NO 1 and having the same function as CAL-A and
- in that the conversion is done in an essentially aqueous medium.

The term "oligo-/polyester" comprises oligoesters with 2-9 repeating units and polyesters with 10 or more repeating units. Further details are disclosed below in the context of Scheme 1. The text below refers to the term "polyester" for oligo-/polyester. In other words, the wording "polyester" as used herein comprises oligoester(s) and polyester(s).

The lactone is preferably selected from formula I, 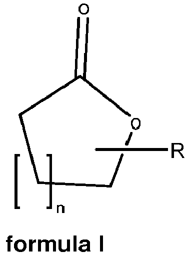 wherein the lactone has at least one substituent R at variable position of the lactone ring (including also the C-atoms within [CH₂]ₙ),
- R: being selected from hydrogen or C1-C20-alkyl, -alkenyl, -alkinyl or C1-C20-cycloalkyl, -cycloalkenyl, -cycloalkinyl or C1-C20-aryl, wherein in case of more than one aliphatic or aromatic ring system the ring systems are condensed or separate and
- n: being selected from 0-17.

Preferably, R is selected from C1-C6-alkyl, more preferably from linear unbranched C1-C4-alkyl, most preferred is R methyl.

Independently, n is preferably selected from 1-9, more preferably from 1-4, most preferred is n = 3 in one preferred embodiment. In another equally preferred embodiment, n is preferably selected from 10-16, more preferably from 12-15, most preferred is n = 12 in this embodiment.

In one embodiment, where R is not hydrogen, at least one substituent R in the lactone of formula I is positioned at the C-atom directly beside the ester-group without a further substituent R at the other C-atom directly beside the ester-group (see formulas Ia or Ib).

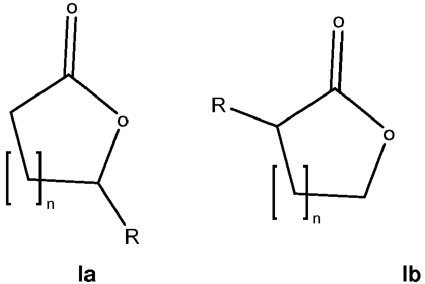

If more than one R≠H is present, preferably one of the C-atoms directly beside the ester-group remains free, i.e. bears only hydrogens.

In one embodiment, the lactone of formula I has preferably at least one C-atom of the cyclic structure replaced by a hetero atom, preferably selected from the 15^{th} or 16^{th} group of the periodic table, more preferably selected from nitrogen, oxygen or sulphur, most preferred nitrogen. The at least one hetero atom, especially the at least one nitrogen atom, may have a substituent R1, which is selected from hydrogen or C1-C20-alkyl, alkenyl, -alkinyl or C1-C20-cycloalkyl, -cycloalkenyl, -cycloalkinyl or C1-C20-aryl, wherein in case of more than one aliphatic or aromatic ring system the ring systems are condensed or separated. The substituent R1 is preferably selected from C1-C6-alkyl, more preferably from C1-C4-alkyl, even more preferred from methyl, ethyl, isopropyl, t-butyl, still more preferred from ethyl or methyl, most preferred is methyl as R1.

The "essentially aqueous medium" means water, which may contain up to 95 % by weight of other solvent(s), preferably up to 20 % by weight of other solvent(s), more preferred 0.1 to 5 % by weight of other solvent(s), the rest of 100 % by weight being water. Most preferred as essentially aqueous medium is water completely free of other solvents. "Solvent(s)" means that one other solvent or a mixture of two or more other solvents are used. The other solvent(s) may be inorganic or organic with the only limitation that the solvents have to be usable in combination with the lipase, which converts the lactone to the polyester. Organic solvent(s) may be selected from water miscible organic solvents and water immiscible organic solvents. Water miscible organic solvents may be DMF, DMSO, methyl ethyl ketone, acetone, THF. Water immiscible organic solvents may be ethers, for example, diethyl ether or diisopropyl ether, toluene, hexane. Further (other) solvents are ionic liquids (salts, which are liquid below 100°C) and deep eutectic mixtures/solvents (mixture of at least two components which form a eutectic with a melting point lower than either of the individual components). Less preferred solvents in combination with the lipase are, for example, esters as they would react at the ester bound. The essentially aqueous medium may optionally contain buffers, for example, Tris-HCl, sodium phosphate buffer or glycine-OH buffer. In a preferred embodiment, the essentially aqueous medium consists of water and optionally other solvent(s) and/or buffer(s). The only further components besides educts and products are the respective enzyme(s) CAL-A (or a homologue enzyme with the same function as CAL-A).

In nature, lipases (EC 3.1.1.3) catalyse the hydrolysis of triglycerides to glycerol and fatty acids. These enzymes are able to hydrolyse a large number of unnatural substrates and therefore have often been used as biocatalysts for such applications (Bornscheuer & Kazlauskas, 2006a; Bornscheuer & Kazlauskas, 2006b; Verger, 1998). Lipases can also be applied for esterifications, but in the presence of water, this serves as nucleophile and hydrolysis becomes the favored reaction. Thus, esterification reactions must be performed in organic media containing only very small amounts of water to maintain the catalytically active form of lipases. Lipases are frequently used as biocatalysts in industrial-scale production of fine chemicals and pharmaceuticals and as additives in household detergents (Verger, 1998).

Lipase CAL-A has been identified from the filamentous fungus *Candida antarctica* (Hoegh et al, 1995). Note that the *Candida antarctica* strain was recently renamed to *Pseudozyma antarctica.* It was found, that CAL-A is highly unusual compared to other lipases and differs considerably in its biochemical properties from CAL-B originating from the same organism (Bosley AJ, 1997; Domínguez de María et al, 2005; Gedey et al, 2001; Liljeblad & Kanerva, 2006). The 3D structure of lipase CAL-A has been elucidated (Ericsson et al, 2008).

A few lipases have been reported to possess acyltransferase activity (Müller et al, 2013; Briand et al, 1994; Briand et al, 1995a; Briand et al, 1995b; Briand et al, 1995c; Riaublanc et al, 1993). For the lipase/acyltransferase from *Candida parapsilosis* it has been shown that this enzyme can catalyze the alcoholysis of various esters in the presence of a large molar excess of water in a biphasic aqueous/lipid reactant medium and at high thermodynamic water activity (aw > 0.9). The hydrolysis of the ester is believed to be inhibited by a proposed competition of the acyl acceptor with water (Briand et al, 1994; Briand et al, 1995a; Briand et al, 1995b; Briand et al, 1995c; Riaublanc et al, 1993). In 2002, this enzyme was successfully cloned and recombinantly produced in *Saccharomyces cerevisiae* (Neugnot et al, 2002). The obtained 465-amino-acid protein sequence contained the consensus motif G-X-S-X-G, which is conserved among lipolytic enzymes. Homology investigations showed that this enzyme has a sequence similarity of 31 % to CAL-A (Müller et al, 2013). Recently, the inventors published regarding CAL-A acyltransferase activity in fatty acid ethyl ester synthesis based on triglyceride and ethanol in an aqueous system (Müller et al, 2014).

The present invention discloses a new method for the ring-opening polymerization of lactones to the corresponding esters as exemplified for ε-CL yielding the corresponding polyester. Surprisingly, it was found that the ring-opening polymerization of the lactone is the only reaction catalysed by lipase CAL-A in this reaction. Despite the presence of a bulk aqueous phase, nearly no hydrolysis of the oligomers to ε-CL and nearly no hydrolysis of the lactone to the free acid was observed. "Nearly no hydrolysis" means less than 2% hydrolysis. In other words, it was found that CAL-A is a lipase which exhibits acyltransferase activity even in an essentially aqueous medium. Normally, a lipase catalyses the hydrolysis of fats (lipids). It is also known that transesterification can be performed by lipases in water-free media. When the system contains water besides ester, alcohol and lipase, the ester is normally transformed into the free fatty acid. Even if several lipases also catalyse the formation of esters resulting in a transesterification via a free acid intermediate, lots of processes are negatively involved by the free acid intermediate. Thus, an enzyme having direct acyltransferase activity, which works in an essentially aqueous medium, is extremely advantageous.

Thus, CAL-A is the most preferred enzyme due to its workability in an essentially aqueous medium and its acyltransferase activity. Instead of CAL-A, a homologue enzyme can be used which has sequence identity of at least 25% as mentioned above, preferably of at least 50%, more preferred of at least 65%, even more preferred of at least 90%, most preferred of at least 95% with SEQ ID NO 1 and has the same function as CAL-A.

"Sequence identity of at least xx% with SEQ ID NO 1" means that one or more amino acid(s) of SEQ ID NO 1 are deleted, replaced or added while the enzyme keeps its function (here and below: "xx" is a placeholder for the individual values as mentioned). Its function, i.e. the "function as CAL-A" means that the enzyme shows at least 50%, preferably at least 75%, more preferred at least 90%, of the lipase/acyltransferase activity, especially acyltransferase activity, of CAL-A in the essential aqueous medium, i.e. converts the lactone at least half as well as CAL-A into the polyester.

In a preferred embodiment, the enzyme is selected from CAL-A and the homologue enzymes listed in Table 1, which also shows the degree of sequence identity of these enzymes.

**Tab. 1: CAL-A and homologue enzymes**

| **SEQ ID NO** | **Abbreviation** | **UniProt/ GenBank** | **Origin** | **Similarity to CAL-A (Sequence Identity)** |
|---|---|---|---|---|
| 01 | CAL-A | D4PHA8 | *Candida antarctica* | 100% |
| 02 | CAL-A_pseu. | M9LN88 | *Pseudozyma antarctica* (strain T-34) | 98% |
| 03 | Lip_K | Q8NIP2 | *Kurtzmanomyces* sp. I-11 | 77% |
| 05 | Lip_PseuHub | R9NZ15 | *Pseudozyma hubeiensis* (strain SY62) | 69% |
| 04 | Lip_UstHor | I2FWQ0 | *Ustilago hordei* (strain Uh4875-4) | 68% |
| 06 | Lip_UM03410 | Q4P903 | *Ustilago maydis* (strain 521) | 67% |
| 07 | Lip_PSEUBRA-1g | EST09784 | *Pseudozyma* sp. GHG001 | 66% |
| 08 | Lip_SpoRei_E7 | E7A3D1 | *Sporisorium reilianum* (strain SRZ2) | 65% |
| 09 | Lip_SpoRei_E6 | E6ZNA3 | *Sporisorium reilianum* (strain SRZ2) | 57% |
| 10 | Lip_PSEUBRA-1g | EST09784 | *Pseudozyma* sp. GHG001 | 56% |
| 11 | CpLIP 2 | Q8NIN8 | *Candida parapsilosis* | 29% |

A preferred sub-group of enzymes is:
- CAL-A (SEQ ID NO 1),
- CAL-A_pseu. (SEQ ID NO 2),
- Lip_UM03410 (SEQ ID NO 6),
- CpLIP 2 (SEQ ID NO 11).

A more preferred embodiment uses the enzymes
- CAL-A (SEQ ID NO 1),
- CAL-A_pseu. (SEQ ID NO 2), with CAL-A being the most preferred enzyme.

The lipase (enzyme) may also be a combination of one or more lipases, especially from the selections described above.

### Cycloalkanones as educts

In order to extend the group of possible educts, the reaction system was extended to cycloalkanones as starting point. This requires the use of a second enzyme, which converts the cycloalkanone to the lactone.

The method for producing polyesters starting from cycloalkanones is characterized that in one vessel (one-pot-reaction) and in an essentially aqueous medium a cycloalkanone is converted with
- a Baeyer-Villiger-Monooxygenase (BVMO) and
- Lipase A from *Candida antarctica* (CAL-A) according to SEQ ID NO 1 or a homologue enzyme having sequence identity of at least 25% with SEQ ID NO 1 and having the same function as CAL-A,
which convert the cycloalkanone (via a lactone) into the polyester.

The essential aqueous medium is generally defined above in the context of the lactone reaction. In this preferred embodiment, the essentially aqueous medium consists of water and optionally solvent(s) and/or buffer(s), for example, Tris-HCl, sodium phosphate buffer or glycine-OH buffer. The only further components besides educts and products are the respective enzyme(s) BVMO, CAL-A (or a homologue enzyme with the same function as CAL-A) and their cofactor(s), for example, NADH/NAD⁺ or NADPH/NADP⁺, and components such as oxygen.

Regarding CAL-A and its homologues, the same applies as outlined above in the context of the lactone reaction.

The method starting from cycloalkanones is regarded as a 2-step-conversion, which is done in one vessel (one-pot-reaction) and in an essentially aqueous medium
- starting from a cycloalkanone, which is converted to a lactone by a Baeyer-Villiger-Monooxygenase (BVMO, step 1), and
- finally, the lactone is converted to the polyester by CAL-A according to SEQ ID NO 1 or a homologue enzyme having sequence identity of at least 25% with SEQ ID NO 1 and having the same function as CAL-A (step 2).

The cycloalkanone is preferably selected from formula II, 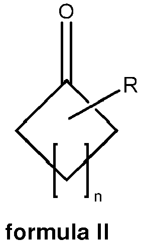 wherein the cycloalkanone has at least one substituent R at variable ring position (including also the C-atoms within [CH₂]ₙ),
- R: being selected from hydrogen or C1-C20-alkyl, -alkenyl, -alkinyl or C1-C20-cycloalkyl, -cycloalkenyl, -cycloalkinyl or C1-C20-aryl, wherein in case of more than one aliphatic or aromatic ring system the ring systems are condensed or separate and
- n: being selected from 0-17.

Preferably, R is selected from C1-C6-alkyl, more preferably linear unbranched C1-C4-alkyl, most preferred methyl.

Independently, n is preferably selected from 1-9, more preferably from 1-4, most preferred is n = 3 in one preferred embodiment. In another equally preferred embodiment, n is preferably selected from 10-16, more preferably from 12-15, most preferred is n = 12 in this embodiment.

The at least one substituent R in the cycloalkanone of formula II is positioned at the C-atom directly beside the C-atom of the keto-group without a further substituent R at the other C-atom directly beside the C-atom of the keto-group. If more than one R≠H is present, preferably one of the C-atoms directly beside the keto-group remains free, i.e. bears only hydrogens.

The cycloalkanone of formula II has preferably at least one C-atom of the cyclic structure replaced by a hetero atom, preferably selected from the 15^{th} or 16^{th} group of the periodic table, more preferably selected from nitrogen, oxygen or sulphur, most preferred nitrogen. The at least one hetero atom, especially the at least one nitrogen atom, may have a substituent R1, which is selected from hydrogen or C1-C20-alkyl, alkenyl, -alkinyl or C1-C20-cycloalkyl, -cycloalkenyl, -cycloalkinyl or C1-C20-aryl, wherein in case of more than one aliphatic or aromatic ring system the ring systems are condensed or separated. The substituent The substituent R1 is preferably selected from C1-C6-alkyl, more preferably from C1-C4-alkyl, even more preferred from methyl, ethyl, isopropyl, t-butyl, still more preferred from ethyl or methyl, most preferred is methyl as R1.

In this first step, cycloalkanone is converted by a Baeyer-Villiger-Monooxygenase (BVMO, e.g. from *Acinetobacter calcoaceticus,* also named cyclohexanone monooxygenase; CHMO) to the lactone. For example, cyclohexanone is converted into ε-CL. The system requires a cofactor NADPH, which is here converted to NADP⁺.

The CHMO from *Acinetobacter calcoaceticus* NCIMB 9871 (EC 1.14.13.22), which was formerly only mentioned as *Acinetobacter sp.,* is the most extensively studied BVMO and the most popular representative for BVMOs of prokaryotic origin (Balke et al, 2012; de Gonzalo et al, 2010). In 1976, this enzyme was discovered and characterized biochemically for the first time (Donoghue et al, 1976). The research with Baeyer-Villiger-Monooxygenases was mostly restricted to two microorganisms, *Acinetobacter calco-aceticus* (Donoghue et al, 1976) and *Pseudomonas putida* (York, 1961) until the mid-1990s. Because the enzyme of *A. calcoaceticus* has a high activity in the conversion of cyclohexanone to ε-caprolactone hence it is usually named cyclohexanone monooxygenase. *A. calcoaceticus* is a pathogenic strain and can only be grown in laboratories with a proper permission (S2 gene technology standard). Stewart *et al.* overcame this limitation by succeeding first in the cloning and functional expression of this CHMO (Kayser & Clouthier, 2006; Stewart, 1998). It has been shown that this enzyme can convert hundreds of different compounds (Stewart, 1998). Also to demonstrate the capability of BVMOs to catalyse other oxidation reactions, this enzyme has been used for sulfoxidations, oxidations of selenium and boron-containing compounds, epoxidations and N-oxidations.

The CHMO from *Acinetobacter calcoaceticus* is the most preferred enzyme regarding the BVMO in the present invention. However, a large diversity of different BVMOs has become available within the recent years and several of them are also able to catalyze the conversion of cycloalkanones to lactones. Thus, the preferred BVMO of the present invention is CHMO from *Acinetobacter calcoaceticus (CHMO, SEQ ID NO 12,* GenBank: BAA86293.1) or a homologue enzyme, which has a sequence identity of at least 25% with SEQ ID NO 12 and has the same function as CHMO.

Regarding sequence identity, as mentioned above at least 25%, preferably at least 50%, more preferred at least 65%, even more preferred at least 90%, most preferred at least 95% sequence identity with SEQ ID NO 12 are meant. "Sequence identity of at least xx% with SEQ ID NO 12" means that one or more amino acid(s) of SEQ ID NO 12 is/are deleted, replaced or added while the enzyme keeps its function. "Its function / the same function as CHMO" means that the CHMO-homologue enzyme shows at least 50%, preferably 75%, more preferred 90%, of the activity of CHMO in the essential aqueous medium, i.e. converts the cycloalkanone at least half as well as CHMO into the lactone.

In a preferred embodiment, the BVMO-enzyme is selected from the enzymes listed in Table 2.

**Tab. 2: CHMO and homologue enzymes**

| SEQ ID NO | Abbreviation | UniProt/ GenBank | Origin |
|---|---|---|---|
| 12 | CHMO | BAA86293.1 | *Acinetobacter calcoaceticus* |
| -- | CAMO | G8H1L8 | *Cylindrocarpon radicicola* |
| -- | CHMO*_{Xantho}* | CAD10801.1 | *Xanthobacter flavus* |
| -- | CHMO*_{Brachy}* | Q5VJE0 | *Brachymonas petroleovorans* |
| -- | CHMO*_{Arthro}* | AAN37479.1 | *Arthrobacter sp.* BP2 |
| -- | CHMO*_{Rhod}* | Q84H73 | *Rhodococcus sp.* Phi1 |
| -- | CHMO*_{Rhod}* | Q84H76 | *Rhodococcus sp.* Phi2 |
| -- | CHMO 1 | Q9FDI4 | *Brevibacterium sp.* HCU |
| -- | CHMO 2 | Q9FDI3 | *Brevibacterium sp.* HCU |
| -- | Cm-BVMO from strain 10D | BAM80902.1 (annotated in GenBank as a steroid monooxy-genase) | *Cyanidioschyzon merolae* |

Since all these enzymes are known and listed in databases as mentioned in Table 2 with their complete amino acid sequences, these sequences beside the sequence of CHMO are not repeated here in detail.

Cyclohexanone monooxygenases from various prokaryotic origins were described until now such as the CHMO from *Arthrobacter* BP2 (CHMO*_{Arthro}*), from *Brachymonas petro-leovorans* (CHMO*_{Brachy}*), 2 from *Rhodococcus sp. (Rhodococcus strain* Phi1, *Rhodococcus strain* Phi2) (CHMO*_{Rhod}*), 2 from *Brevibacterium sp.* HCU⁹ and from *Xanthobacter flavus* (CHMO*_{Xantho}*).

The *Xanthobacter flavus* BVMO shares 57% sequence identity to the *Acinetobacter sp.* NCIMB 9871 BVMO. The *Comamonas* BVMOs is also closely related to the cyclohexanone monooxygenase of *Acinetobacter sp.* NCIMB 9871 (up to 64% sequence identity).

The two *Brevibacterium* cyclohexanone monooxygenases are part of the large family of flavin-dependent monooxygenases. Sequences with the highest similarity are the *Acinetobacter* cyclohexanone monooxygenase and the *Rhodococcus* steroid monooxygenase (GenBank: AB010439.1) with amino acid identity between 35 and 40%. The significance of the two isozymes of the cyclohexanone monooxygenase is not known, although it may be related to their different substrate specificities.

The monooxygenases from the two *Rhodococcus* species are relatively similar and share 90% amino acid identity and 89% nucleotide identity. The *Arthrobacter* enzyme is more distantly related, with 84% amino acid identity to both the *Rhodococcus* strain Phi1 and *Rhodococcus* strain Phi2 enzymes.

The Cyclopentanone monooxygenase (CPMO) from *Pseudomonas* NCIB 9872 was first described in 1972 and converts cyclohexanone with nearly the same rate as the preferred substrate cyclopentanone. The cyclopentanone monoxygenase (CPMO*_{Coma}*) from *Comamonas sp.* NCIMB 9872 was successfully identified, cloned and expressed in 2003 by van Beilen et al. in 2002 and is also able to convert cyclohexanone to the corresponding lactone. The 3.8 kb sequence obtained from *Comamonas sp.* NCIMB 9872 encoded a protein with 36% sequence identity to the *Acinetobacter sp.* NCIMB 9871 BVMO, and the N-terminus of the predicted protein (named CpnB) was identical to the published N-terminus of cyclopentanone monooxygenase.

The first BVMO from a eukaryotic origin to be cloned and expressed is the cycloalkanone monooxygenase (CAMO) from *Cylindrocarpon radicicola* ATCC 11011 (identical to *Cylindrocarpon destructans* DSM 837) and was first described in 2012 (Leipold et al. (2012). This enzyme shows a high activity against cyclohexanone with a specific activity of 4.22 mg/ml. The 531-residue primary structure of the novel BVMO revealed 46.4% sequence identity to the CHMO from *Rhodococcus sp.* Phi1 and 44.1 % compared to the CHMO from *Acinetobactersp.* NCIB 9871.

Recently the Fraaije group discovered a further BVMO from a photosynthetic eukaryote in 2013. This type I BVMO from the red alga *Cyanidioschyzon merolae* (Cm) shows a good activity against cyclohexanone for the conversion to ε-caprolactone. The primary structure of the translated protein shares 43% identity with the sequence of PAMO, 37% with cyclohexanone monooxygenase (CHMO) from *Acinetobacter* NCIMB 9871 and 50% with CHMO from *Rhodococcus* Phi1.

The BVMO may also be a combination of two or more BVMOs from the above-mentioned selection.

### Cycloalkanoles as educts

In order to extend the educt group further, cycloalkanols were used as starting point.

The method for producing polyesters starting from cycloalkanols is characterized in that in one vessel (one-pot-reaction) and in an essentially aqueous medium
a cycloalkanol is converted with
- an alcohol dehydrogenase (ADH),
- a Baeyer-Villiger-Monooxygenase (BVMO) and
- Lipase A from *Candida antarctica* (CAL-A) according to SEQ ID NO 1 or a homologue enzyme having sequence identity of at least 25% with SEQ ID NO 1 and having the same function as CAL-A,
which convert the cycloalkanol into the polyester. Scheme 1 below gives a general overview.

Further advantages of this new method are an improved E-factor (relation of amount of waste, which is generated [kg/process], to amount of product [kg/process], i.e. amount of waste/amount of product (Sheldon, 1992)) compared to chemical or multistep bio catalytic processes, i.e. the saving of organic solvents due to the "one-pot-process" as well as saving of energy costs because regeneration of intermediate products on the way from cycloalkanol to polyester is obsolete.

The essentially aqueous medium is defined as described above with respect to the lactone reaction. In this preferred embodiment, the essentially aqueous medium consists of water and optionally solvent(s) and/or buffer(s), for example, Tris-HCl, sodium phosphate buffer or glycine-OH buffer. The only further components besides educts and products are the respective enzyme(s) ADH, BVMO, CAL-A (or a homologue enzyme with the same function as CAL-A) and their cofactor(s), for example, NADH/NAD⁺ or NADPH/NADP⁺, and components such as oxygen.

Regarding CAL-A and its homologues, the same applies as outlined above in the context of the lactone reaction.

The method is regarded as a 3-step-conversion done in one vessel (one-pot-reaction) and in an essentially aqueous medium
- starting from a cycloalkanol, which is initially converted to a cycloalkanone by an alcohol dehydrogenase (ADH, step 0) and
- subsequently the cycloalkanone is converted to a lactone by a Baeyer-Villiger-Monooxygenase (BVMO, step 1), and
- finally, the lactone is converted to the polyester by Lipase A from *Candida antarctica* (CAL-A) according to SEQ ID NO 1 or a homologue enzyme having sequence identity of at least 25% with SEQ ID NO 1 and having the same function as CAL-A
   (step 2).

In a preferred embodiment, the cycloalkanol is selected from formula III, 3 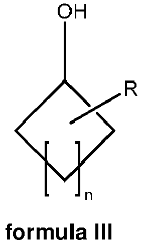 wherein the cycloalkanol has at least one substituent R at variable ring position (including also the C-atoms within [CH₂]ₙ),
- R: being selected from hydrogen or C1-C20-alkyl, alkenyl, -alkinyl or C1-C20-cycloalkyl, -cycloalkenyl, -cycloalkinyl or C1-C20-aryl, wherein in case of more than one aliphatic or aromatic ring system the ring systems are condensed or separate and
- n: being selected from 0-17.

Preferably, R is selected from C1-C6-alkyl, more preferably linear unbranched C1-C4-alkyl, most preferred methyl.

Independently, n is preferably selected from 1-9, more preferably from 1-4, most preferred is n = 3 in one preferred embodiment. In another equally preferred embodiment, n is preferably selected from 10-16, more preferably from 12-15, most preferred is n = 12 in this embodiment.

In one embodiment, where R is not hydrogen, the at least one substituent R in the cycloalkanol of formula III is positioned at the C-atom directly beside the C-atom with the hydroxy-group without a further substituent R at the other C-atom directly beside the C-atom with the hydroxy-group. If more than one R≠H is present, preferably one of the C-atoms directly beside the hydroxy-group remains free, i.e. bears only hydrogens.

The cycloalkanol of formula III has preferably at least one C-atom of the cyclic structure replaced by a hetero atom, preferably selected from the 15^{th} or 16^{th} group of the periodic table, more preferably selected from nitrogen, oxygen or sulphur, most preferred nitrogen. The at least one hetero atom, especially the at least one nitrogen atom, may have a substituent R1, which is selected from hydrogen or C1-C20-alkyl, alkenyl, -alkinyl or C1-C20-cycloalkyl, -cycloalkenyl, -cycloalkinyl or C1-C20-aryl, wherein in case of more than one aliphatic or aromatic ring system the ring systems are condensed or separated. The substituent The substituent R1 is preferably selected from C1-C6-alkyl, more preferably from C1-C4-alkyl, even more preferred from methyl, ethyl, isopropyl, t-butyl, still more preferred from ethyl or methyl, most preferred is methyl as R1.

Regarding special embodiments, the cycloalkanol can be selected from formula IIIa, IIIb or IIIc, 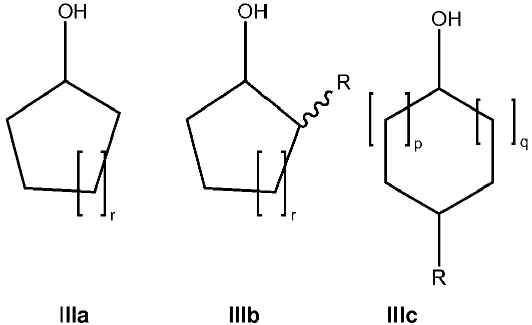 wherein R has the same meaning as explained above (R=H in IIIa), r corresponds to n from above -1, i.e. r=-1 to 16, and p, q in IIIc are identical integers (p=q) with the condition that the sum p+q is 0 or an even integer selected from 2-16.

As mentioned above for n, there are two alternative embodiments also for r: Independently, r is preferably selected from 0-8, more preferably from 0-3, most preferred is r = 2 in one preferred embodiment. In another equally preferred embodiment, r is preferably selected from 9-15, more preferably from 11-14, most preferred is n = 11 in this embodiment.

For the oxidation of alcohols, so-called oxidoreductases (EC. 1.x.x.x) are the catalysts of choice (Drauz et al., 2012). Within this diverse class of enzymes, an alcohol dehydrogenase (ADH, EC 1.1.1.x, also often named ketoreductase, KRED) was chosen for this special task. Alcohol dehydrogenases in general catalyse the reversible hydride abstraction from the substrate and simultaneous transfer to the oxidized nicotinamide cofactor NAD(P)⁺. This class of enzymes is endogenously found in all kingdoms of life. Until now, 85 ADHs with specificity for NADP⁺ and 261 ADHs with specificity for NAD⁺ are known (http://www.brenda-enzymes.org). The most popular, commercially available ADHs originate from horse liver (HLADH) and various microorganisms, such as *Thermoanaerobium brockii* (TBADH), baker's yeast (YADH), *Candida,* and *Lactobacillus kefir* (Lk-ADH) (Hummel, 1997; Kroutil et al, 2004; Reid, 1994). The ADH from *Lactobacillus kefir* was first cloned, expressed and characterized 2006 by Weckbecker (Weckbecker & Hummel, 2006).

The preferred alcohol dehydrogenase of the present invention is ADH from *Lactobacillus kefir (*Lk-ADH, SEQ ID NO 13), which converts the cycloalkanol to cycloalkanone under consumption of the cofactor NADP⁺ to yield NADPH, or a homologue enzyme, which has a sequence identity with Lk-ADH of at least 25% with SEQ ID NO 13 and has the same function as Lk-ADH.

Regarding sequence identity, as mentioned above at least 25%, more preferred at least 50%, even more preferred at least 65%, still more preferred at least 90%, most preferred at least 95% sequence identity with SEQ ID NO 13 are meant. "Sequence identity of at least xx% with SEQ ID NO 13" means that one or more amino acid(s) of SEQ ID NO 13 iis/are deleted, replaced or added while the enzyme keeps its function. "Its function / the same function as Lk-ADH" means that the Lk-ADH-homologue enzyme shows at least 50%, preferably 75%, more preferred 90%, of the activity of Lk-ADH in the essential aqueous medium, i.e. converts the cycloalkanol at least half as well as Lk-ADH into the cycloalkanone.

In a preferred embodiment, the Lk-ADH -homologue enzyme is selected from the enzymes listed in Table 3.

**Tab. 3: Lk-ADH and homologue enzymes**

| SEQ ID NO | Abbreviation | UniProt/ GenBank | Origin |
|---|---|---|---|
| 13 | Lk-ADH | AY267012. 1 | *Lactobacillus kefir* |
| -- | RR-ADH | CAD36475. 1 | *Rhodococcus ruber* |
| -- | TBADH | CAA46053. 1 | *Thermoanaerobium brockii* |
| -- | Ss-ADH | P39462 | *Sulfolobus solfataricus* |
| -- | Ap-ADH | Q9Y9P9 | *Aeropyrum pernix* |

Since all these enzymes are known and listed in databases as mentioned in Table 3 with their complete amino acid sequences, these sequences are not repeated here in detail.

Except of the ADH from *Lactobacillus kefir,* some more ADHs from various origins are able to convert cyclohexanol to cyclohexanone such as the ADHs from *Thermoanaerobium brockii* (TBADH), *Rhodococcus ruber* (RR-ADH) *Sulfolobus solfataricus* (Ss-ADH) and *Aeropyrum pernix* (Ap-ADH).

Of the numerous medium-chain microbial ADHs, which are all homotetramers, mainly the enzymes from thermophilic and hyperthermophilic bacteria were comprehensively biochemically characterized. From this group the three-dimensional structures of the enzymes from *Thermoanaerobium brockii, Aeropyrum pernix* and *Sulfolobus solfataricus* are known.

Also from mesophilic microorganisms ADHs were isolated and characterized, such as ADHs from *Rhodococcus* strains.

Based on the amino acid sequences the thermophilic or hyperthermophilic ADHs show homologies between 40-85% to the mesophilic enzymes with overlapping three-dimensional structures and similar reaction mechanisms; among each other enzymes from thermophilic and hyperthermophilic origin show very high similarities of more than 90%.

All these enzymes are characterized by their coenzyme specificity (NAD⁺ or NADP⁺) and their often-limited substrate scope, with a preference for aliphatic, cyclic or open chain side chains or aromatic structures there.

The ADH may also be a combination of two or more ADHs, especially from the above-mentioned selections.

The complete cycloalkanol-based system is composed of three enzymes: first, cycloalkanol is converted by an alcohol dehydrogenase (e.g., from *Lactobacillus kefir,* Lk-ADH) to cycloalkanone (step 0) under consumption of the cofactor NADP⁺ to yield NADPH. In the next step, cycloalkanone is converted by a Baeyer-Villiger-Monooxygenase (BVMO, e.g. from *Acinetobacter calcoaceticus,* also named cyclohexanone monooxygenase; CHMO) to the lactone (step 1).

The cofactor NADPH is here converted to NADP⁺ hence providing a self-sufficient cofactor recycling. This reaction has been described (Mallin et al, 2013; Staudt et al, 2013a). The final new reaction step is the ring-opening polymerization catalyzed by the lipase (e.g., lipase CAL-A, Novozymes, step 2) in the presence of a bulk aqueous system. The complete reaction is shown in Scheme 1.

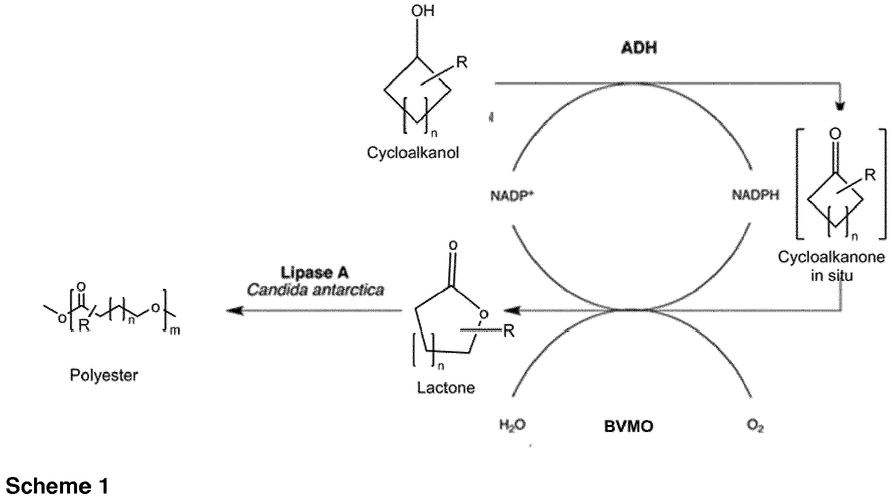

Scheme 2 shows a special embodiment of the invention starting from cyclohexanol.

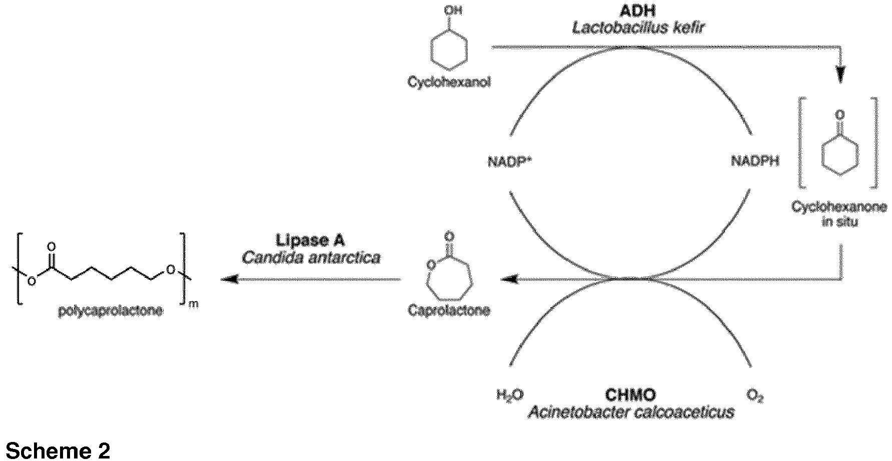

The oxidation of cyclohexanol (CHL) with the cofactor NADP⁺ catalyzed by the alcohol dehydrogenase (Lk-ADH) yields cyclohexanone (CHO) and the reduced cofactor NADPH. The oxygen-dependent cyclohexanone monooxygenase (CHMO) converts this ketone to ε-caprolactone (ε-CL) and the oxidized form of the cofactor is formed; the latter being reused in the next catalysis cycle. The ε-CL is directly converted by CAL-A to polycaprolactone in the aqueous system.

The final polyester as shown in Schemes 1 and 2 comprises polyesters with ten or more units (m≥10) as well as oligoesters with two to nine units (m = 2-9). The preferred degree of polymerisation is 2 to 20 (m = 2-20), more preferred 8 to 15 units (m = 8-15).

### Advantages

Surprisingly, it was found that the ring-opening polymerization of the lactone is the only reaction catalysed by lipase CAL-A in this 3-enzyme cascade reaction starting from the cycloalkanol. Despite the presence of a bulk aqueous phase, nearly no hydrolysis of the lactone to the corresponding acid was observed. "Nearly no hydrolysis" means less than 2% hydrolysis. The same applies to the 2-enzyme cascade reaction starting from the cycloalkanone and to the 1-enzyme reaction starting from the lactone itself.

Current enzymatic processes to make lactones such as ε-CL are substantially hampered by strong product and substrate inhibition as detailed for the CHMO (Staudt, Burda et al. 2013). Substrate inhibition can be reduced by slow feeding of the substrate into the reaction system. In contrast, the problem of product inhibition is in general difficult to solve. Reaction engineering principles include continuous precipitation or removal of the product by e.g. distillation or evaporation. Both methods cannot be applied for lactones such as ε-CL. Furthermore, reaction conditions must be carefully chosen to avoid hydrolysis of ε-CL to the 6-hydroxycaproic acid as the free acid cannot easily be used as monomer for polymerization. The very high water solubility of lactones such as ε-CL excludes product removal by extraction. One alternative is the use of adsorption resins (to achieve so-called in situ product removal) (Baldwin et al, 2008; Geitner et al, 2010). Unfortunately, no adsorption resin could be yet identified with high adsorption selectivity for the lactone, but little adsorption of the ketone substrate. Furthermore, such a method causes extra costs for the resin, laborious washing steps to release product and larger equipment volume to compensate for the space required for the resin.

The new process of the present invention here hence not only enables an efficient direct in situ polymerization of lactones to polyesters, but also overcomes the problem of product inhibition. Furthermore, the lipase-catalyzed ROP can be performed directly in the presence of an ADH and a BVMO in a 3-enzyme cascade reaction in an aqueous reaction system. The same applies to the 2-enzyme cascade reaction starting from the cycloalkanone, where the lipase-catalyzed ROP can be performed directly in the presence of a BVMO in a 2-enzyme cascade reaction in an aqueous reaction system. In each case, a one-pot-reaction in an essentially aqueous medium is possible.

Notably, in current processes, the enzymatic production of lactones such as ε-CL by using BVMO alone or in combination with an ADH cannot be performed as a one-pot process as the lactone must be separated and can only be polymerized (chemically or enzymatically using the lipases described above) in a second reaction step in the neat lactone or the presence of organic solvents.

### Description of Figures

- **Fig. 1:**: PCL produced by a biocatalysis reaction starting from 1 M ε-CL and using lipase CAL-A in distilled water. After 2 days incubation at 4°C a white sediment was observed that represents the crystallized polycaprolactone.
- **Fig. 2:**: Comparison of the obtained ε-CL yields in biocatalysis reactions with Lk-ADH and CHMO with and without addition of CAL-A. The ε-CL yield is given in concentration [mM]; the grouped columns represent the different substrate concentrations from 0 to 100 mM CHL. The hatched columns show the obtained ε-CL concentrations without and the white columns the ε-CL concentrations with addition of CAL-A.

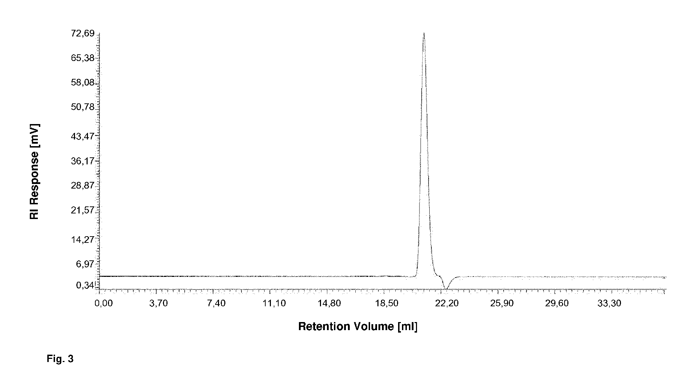

- **Fig. 3:**: GPC chromatogram for analysis of ε-CL as monomeric standard (molar mass: 114.14 g/mol). The observed single narrow peak represents the internal standard toluene.

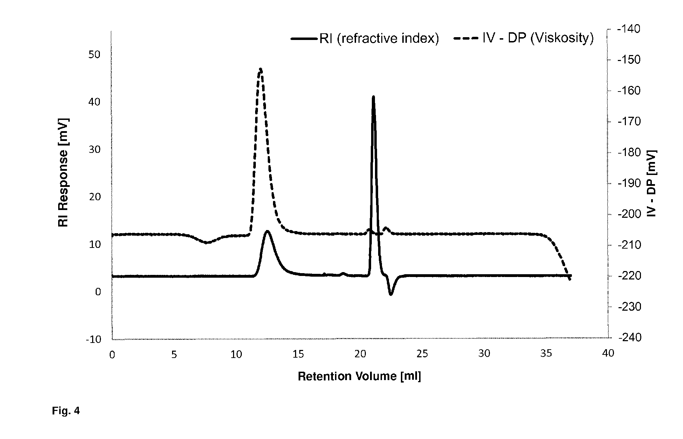

- **Fig. 4:**: GPC chromatogram for analysis of polycaprolactone as polymer standard. The observed single narrow peak represents the internal standard toluene; the peak at a retention volume of 12.8 ml represents the poly-caprolactone.
- **Fig. 5:**: GPC chromatogram of the analysis of synthesized polycaprolactone by CAL-A extracted from a biocatalysis approach. The observed single narrow peak represents the internal standard toluene; the various peaks between retention volumes of 14.80 to 20.3 ml show the formed polycaprolactone of varying sizes.
- **Fig. 6:**: Standard curve for calculation of the molecular weight of the polycaprolactone.

Below, the method(s) is/are described by means of examples without limiting the invention thereby.

### EXAMPLES

### 2. Material and Methods

### 2.1 Chemicals

Unless stated otherwise all chemicals were purchased from Sigma-Aldrich (Steinheim, Germany), Fluka (Buchs, Switzerland) and Merck (Darmstadt, Germany). Lyophilized Chirazyme^{®} L-5 (CAL-A) (1 MU/26,6 g lyophilisate) was obtained from Roche (Penzberg, Germany). The activity of Chirazyme^{®} L-5 was specified as indicated by the supplier with > 30 U/mg_{Lyo} (determined with 100 mM tributyrin in 10 mM potassium phosphate buffer with an pH of 7.0 at 25°C). *Bacillus subtilis* esterase 2 (Henke et al, 2002; Schmidt et al, 2007) was produced by Enzymicals (Greifswald, Germany). Activity of esterase BS2 was specified as indicated by the supplier with >5 U/mg_{Lyo} determined using a stock solution of *p*-nitrophenyl acetate (30 mM in DMSO) in sodium phosphate buffer (50 mM, pH 7.5) with a final DMSO concentration of 10% (in the cuvette).

### 2.2 Strains and culture conditions

The expression system comprising ADH and CHMO was made as described (Mallin et al, 2012; Staudt et al, 2013b). Briefly, the gene encoding CHMO of *Acinetobacter calcoaceticus* was readily cloned into pET28a(+) in frame with a N-terminal His₆-tag. The gene of ADH from *Lactobacillus kefir* was cloned into the same vector behind the gene of CHMO from *Acinetobacter calcoaceticus* in frame with an additional ribosome-binding site and without any tag. This construct was transformed into *E. coli* BL21 (DE3). The enzymes were expressed together in *E. coli* BL21 (DE3) cells grown at 20°C in shaking flasks. CHMO and ADH expression was simultaneously induced with 1.0 mM IPTG at an OD₆₀₀ of 0.7 at 20°C and cells were grown in terrific broth media. After expression for 5-6 h cells were harvested (centrifuged at 1344 g at 4°C for 15 min), washed twice in sodium phosphate buffer (50 mM, pH 8.0). The bacterial cell pellet was resuspended in the same buffer to give a wet cell dry of 100 g/L. This cell suspension was stored over night with 0.01 % DMSO for cell permeabilization and then was used as resting cells in biocatalysis reactions.

### 2.3 pH-STAT experiments

All measurements were done with the 877 Titrino Plus from Metrohm (Filderstadt, Germany).

Using the pH-STAT, the autohydrolysis of ε-CL at different temperatures and pH values was determined. Furthermore, it was investigated whether CAL-A is able to hydrolyze ε-CL in an aqueous reaction system. The *Bacillus subtilis* esterase 2 (BS2) served as positive control in the hydrolysis of ε-caprolactone to 6-hydroxycaproic acid leading to a measureable pH change in the pH-STAT.

### 2.3.1 Determination of autohydrolysis of ε-CL

For the determination of autohydrolysis, 100 mM ε-CL (Sigma-Aldrich, St- Louis, United States; 97 % purity) were dissolved in 20 ml Tris-HCl buffer (20 mM, pH 7.5) or 20 ml distilled water. The measurement was carried out at 25°C and 30°C over 15 min. For titration, 0.1 N NaOH was used to maintain the pH value constant.

### 2.3.2 Determination of specific activity of esterase BS2 against ε-CL

To determine the enzyme activity of BS2 experiments were performed at 25°C with ε-CL (100 mM final conc.) in 20 ml Tris-HCL buffer (20 mM, pH 7.5) or distilled water. The reaction was started by addition of 100 µL enzyme solution (10 mg/ml BS2 lyophilisate dissolved in Tris-HCl buffer (20 mM, pH 7.5) or distilled water. For titration, 0.1 N NaOH was used.

### 2.3.3 Determination of hydrolysis of ε-CL by CAL-A

This was performed as described above in Section 2.3.1. The reaction was started by addition of 25 µL enzyme solution (100 mg/ml lyophilized CAL-A dissolved in 20 mM Tris-HCl buffer of pH 7.5 or distilled water).

### 2.4 Ring-opening polymerization catalyzed by CAL-A

To verify whether CAL-A can be used for the ring-opening polymerization in an aqueous reaction system, 10 ml distilled water, ε-CL (1 M final conc.) and 100 µl of an enzyme solution (100 mg/ml lyophilized CAL-A dissolved in distilled water) were mixed in a 100 ml shaking flask. The shake flask was closed with a breathable film (AeraSeal film, Excel Scientific, Victorville, United States), shaken with 120 rpm in an Multitron Standard Shaker (Infors HT, Einsbach, Germany) for 30 h at 30°C.

As a control, a reaction batch with CHL instead of ε-CL as substrate, and otherwise identical composition, was used. CHL was used as control since it can be excluded as CAL-A substrate, but is used in the biocatalysis with ADH, CHMO and CAL-A.

A characteristic of polycaprolactone is its crystallization at 4°C. In contrast, the melting point of ε-CL is -1°C. For that reason, the reaction batch was incubated at 4°C overnight to allow the crystallization of the polycaprolactone.

### 2.5 Combined biocatalysis reactions without addition of CAL-A

These biotransformations with ADH and CHMO catalysts were carried out in 100 ml shaking flasks containing 9 ml sodium phosphate buffer (50 mM, pH 8.0), 1 ml cell suspension (100 g/L wet cell dry) prepared as described above in Section 2.2 and different concentrations of CHL (0, 20, 40, 60, 80, 100 mM). The shake flask was closed with a breathable film (AeraSeal film, Excel Scientific, Victorville, United States), shaken in an Multitron Standard Shaker (Infors HT, Einsbach, Germany) with 120 rpm for 24 h at 25°C and 500 µl samples were taken periodically. Samples were extracted with 500 µl dichloromethane containing 2 mM acetophenone as internal standard. Determination of CHL, CHO and ε-CL concentrations was carried out by gas chromatography using a Shimadzu GC-14A equipped with a Hydrodex^{®}-ß3P column (25 m x 0.25 mm, Macherey & Nagel, Düren, Germany) and flame-ionization detection (FID). First, the oven temperature was kept at 60°C for 10 min, followed by an increase to 160°C with a heating rate of 10°C/min. Finally, the temperature was held at 160°C for 10 min. Retention times are: CHL = 15.9 min, CHO = 13.7 min, ε-CL = 20.0 min). The conversion was determined by standard curves (calibration curves) for CHL, CHO and ε-CL.

### 2.6 Combined biocatalysis reactions with addition of CAL-A

These biotransformations comprised all three enzymes (ADH, CHMO, CAL-A) and were carried out in shaking flasks containing 8 ml sodium phosphate buffer (50 mM, pH 8.0), 1 ml cell suspension (100 g/L wet cell dry) prepared as described above in Section 2.2, 1 ml enzyme solution (10 mg/ml lyophilized CAL-A in sodium phosphate buffer) and different concentrations of CHL (0, 20, 40, 60, 80, 100 mM). The shake flask was closed with a breathable film (AeraSeal film, Excel Scientific), shaken with 120 rpm for 65 h at 25°C and 500 µl samples were taken periodically. Samples were extracted with 500 µl dichloromethane containing 2 mM acetophenone as internal standard. Determination of CHL, CHO and ε-CL concentrations was carried out by gas chromatography using a Shimadzu GC-14A equipped with a Hydrodex^{®}-ß-3P column (25 m x 0.25 mm, Macherey & Nagel, Düren, Germany) and flame-ionization detection (FID). First, the oven temperature was kept at 60°C for 10 min, followed by an increase to 160°C with a heating rate of 10°C/min. Finally, the temperature was held at 160°C for 10 min. Retention times are: CHO = 13.7 min, CHL = 15.9 min, ε-CL = 20.0 min). The conversion was determined by standard curves for CHL, CHO and ε-CL.

### 2.7 Gel permeation chromatography

Gel permeation chromatography (GPC) was used to determine the molecular weight (Mw) of the polycaprolactone formed. For the GPC measurements, a system consisting of a LC-20AT pump (Shimadzu, Kyoto, Japane), a auto sampler 3200 (Knauer, Berlin, Germany), a GMHHR-L mixed bed column (Viskotek, Waghäusel, Germany) in series with a PLgel 3_MIXED-E 30 K column (Latek, Eppelheim, Germany), a K2301 refractivity detector (Knauer, Berlin, Germany) and a 270 dual detector (Viscotek, Waghäusel, Germany) were used.

Tetrahydrofuran (THF) was used as solvent and as developing solvent with a flow rate of 1 ml/min. A universal calibration with 12 closed polystyrene standards (66000, 28000, 17600, 12600, 9130, 6100, 4920, 3470, 2280, 1250, 682 and 162 g/mol) (Polymer Standard Devices, Germany) was used. Toluene was the internal standard.

From biotransformations 500 µL samples were taken. These were extracted three times with 500 µL dichloromethane. After evaporation of the solvent, 5 mg of the extracted sample were dissolved in 800 µL tetrahydrofuran (THF). After filtration, 3 µL toluene as internal standard were added. For the measurement, 300 µl of the prepared sample were loaded onto the column. The flow rate was adjusted to 1 ml/min (THF).

The analysis of the chromatogram was carried out using a standard curve of the retention volume

For determination of Mw, the integration area along the retention volume in the chromatogram in which all the polymer peaks are located, was fixed. The type of Mw is an average of all the polymers formed. Mn represents the distribution and Mw/Mn obtained the PDI (polydispersity index), which indicates how polymers were uniformly formed.

### 3. Results

### 3.1 Experiments with the pH-STAT

First, the autohydrolysis of ε-CL (Scheme 3) was determined in distilled water as well as in buffer at 25°C and 30°C (Incubation of ε-CL can result in the formation of the corresponding 6-hydroxy-caproic acid either by autohydrolysis or catalyzed by the esterase BS2).

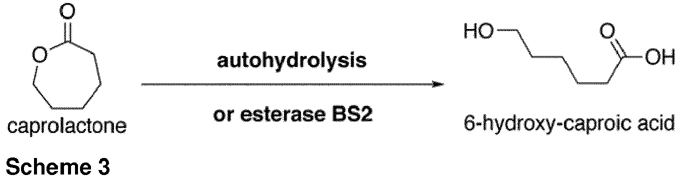

Table 4 shows the hydrolysis values calculated in µmol/min at 25°C and 30°C in distilled water and in a buffered solution. The autohydrolysis of ε-caprolactone is much lower in distilled water and in buffer at 25°C compared to the hydrolysis at 30°C.

In addition to the hydrolysis rate of ε-CL, also conversions are given. This conversion represents the amount of substrate in percent, with hydrolysis over the whole reaction time (based on 100 mM ε-CL substrate concentration as 100 %).

**Table 4: Autohydrolysis in distilled water and Tris-HCl buffer at 25 and 30 °C.**

| | **[µmol/min] at 25 °C** | **Conversion [%]** | **[µmol/min] at 30 °C** | **Conversion [%]** |
|---|---|---|---|---|
| **Distilled water (pH 7.0)** | 0.104 ± 0,00208 | 0.094 ± 0,00187 | 1.1194 ± 0,00101 | 1.075 ± 0,00090 |
| **Tris-HCl buffer (pH 7.5)** | 0.103 ± 0,00265 | 0.093 ± 0,00238 | 0.17 ± 0,01803 | 0.153 ± 0,01622 |

With addition of CAL-A under the same reaction conditions, nearly identical values for hydrolysis, i.e. less than 2 % hydrolysis, in the buffered solution were achieved (see table 5). The pH change in distilled water is slightly higher at 25°C. Also, a slight increase of the conversions in distilled water at 25°C and in the buffered solution at 30 °C was observed.

**Table 5: Titration in distilled water and Tris-HCl buffer at 25 and 30°C with addition of CAL-A.**

| | **[µmol/min] at 25 °C** | **Conversion [%]** | **[µmol/min] at 30 °C** | **Conversion [%]** |
|---|---|---|---|---|
| **Distilled water (pH 7.0)** | 0.32 ± 0,02646 | 0.288 ± 0,02381 | 1.111 ± 0,00503 | 0.999 ± 0,00453 |
| **Tris-HCl buffer (pH 7.5)** | 0.11 ± 0,02165 | 0.102 ± 0,01949 | 0.20 ± 0,02499 | 0.180 ± 0,02249 |

The slight increase in the pH change caused by the addition of CAL-A to a solution of ε-caprolactone could be due to the hydrolysis of ε-caprolactone to the corresponding 6-hydroxy caproic acid or due to the polymerization of ε-CL catalyzed by CAL-A. As we assumed, oligomers are the product of the CAL-A-catalyzed reaction. This means, that with each oligomer, which is formed by CAL-A an acid group (-COOH) is formed. This acid groups lead to a slight pH change, which is detectable by pH STAT.

The specific activity of esterase BS2 against ε-CL was determined with 1,36 U/mg. This value correlates with the activity of other esterases against ε-CL (Henke & Bornscheuer, 2002).

As the calculated slopes of the titration curve are almost identical between autohydrolysis and the experiment in the presence of CAL-A, this indicates that CAL-A is not able to catalyze the hydrolysis of ε-CL to 6-hydroxycaproic acid.

### 3.2 Synthesis of PCL with CAL-A

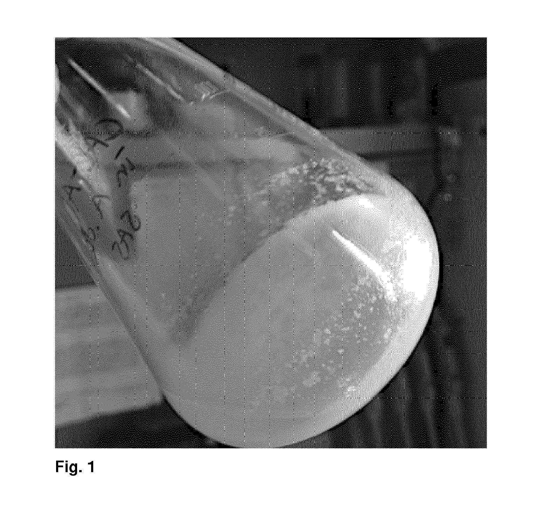

After incubation of CAL-A with ε-CL for 48 h in an aqueous solution, a streaking and also a milky white cloudiness of the solution was observed (Figure 1). This is a clear indication for the polymerization reaction. The control reaction with cyclohexanol instead of ε-CL did not result in a cloudiness. Due to the ability of polycaprolactone to crystallize at 4°C and the lower melting point of ε-CL (-1°C), the incubation of the reaction solution at this low temperature lead to the formation of a white, crystalline precipitate. The longer the incubation at 4°C, the stronger was the formation of the precipitate.

### 3.3 Biocatalysis with Lk-ADH, CHMO and addition of CAL-A

The next step was to examine whether the polymerization of ε-CL by CAL-A still works in biotransformations using resting cells containing Lk-ADH and CHMO.

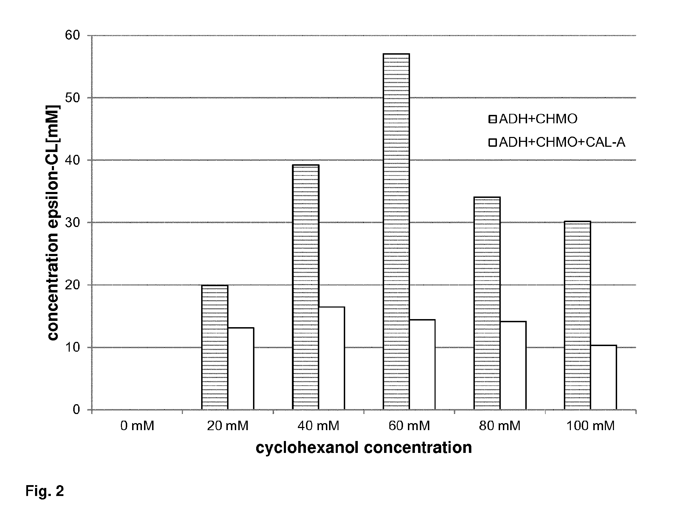

At first, biotransformations with Lk-ADH and CHMO using different cyclohexanol concentrations were performed. This gave 99% and 98% conversion to ε-CL formation at 20 and 40 mM CHL concentration, respectively (Figure 2). With 60 mM CHL 95% conversion could be achieved. At higher substrate concentrations of 80 and 100 mM, the conversion dropped to 42 and 30%, respectively. These conversions were obtained after 24 h of biocatalysis (see Table 6). In Table 6, also the obtained conversions at different substrate concentrations for free enzymes are listed (Staudt et al, 2013a). These results are comparable to the results obtained with whole-cells in biocatalysis reactions.

**Table 6: Comparison of the conversions obtained in biotransformations with Lk-ADH and CHMO.**

| | **conversion [%]** | | |
|---|---|---|---|
| **Substrate concentration** | **CHMO (whole cell bio-catalysis)** | **LK-ADH+CHMO (whole-cell bio-catalysis)** | **Lk-ADH+CHMO (free enzymes) (Staudt, 2013)** |
| **20 mM** | 96 | 99 | 97 (99) |
| **40 mM** | 92 | 98 | 97 |
| **60 mM** | 91 | 95 | 94 |
| **80 mM** | 36 | 42.5 | 36 |
| **100 mM** | 13 | 30 | 24 |

Performing the same reaction under identical reaction conditions, but with the addition of CAL-A, a strong decrease of ε-CL concentration was observed (Figure 2). With 1 mg/ml (per batch) CAL-A during biocatalysis a conversion of 65% and 41 % with 20 and 40 mM CHL, respectively, could be obtained.

Furthermore, this example demonstrates that biotransformations can be performed with all three enzymes (Lk-ADH, CHMO and CAL-A) in a single reaction.

Previous studies have shown, that the CHMO is strongly inhibited by high substrate concentrations, but mostly by high product concentrations (Mallin et al, 2012; Staudt, 2013). The direct polymerization of ε-CL during the reaction represents a new, elegant and simple way to solve the problem of product inhibition. This allows much higher product concentrations and thus makes an industrial applicable process feasible.

### 3.4 Determination of molecular weight of the synthesized PCL

By using gel permeation chromatography, the determination of molecular weight of the synthesized polymer is possible. To determine the molecular weight, the polymer synthesised by CAL-A in a reaction with 1 M ε-CL was used.

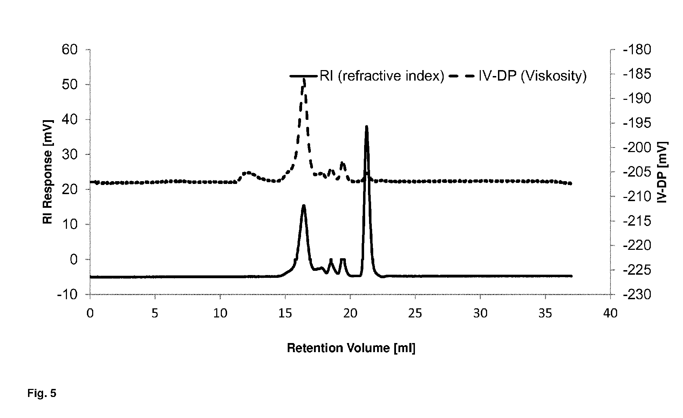

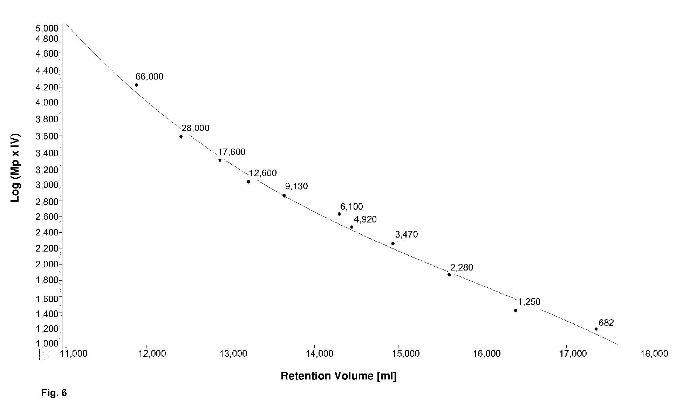

The obtained chromatogram shows several narrow peaks and one broad peak (Figure 5). This suggests that the formed polymer is a mixture of different polymer chains varying in size and thus in the molecular weight. The average of the achieved molecular weight for the synthesized polymer was 1200 g/mol (Figure 6). The molar mass of one ε-CL molecule is 114,14 g/mol. Therefore, the obtained PCL consists of 10 to 11 molecules ε-CL.

The GPC analysis provided the proof for a polymerization reaction catalyzed by CAL-A. In addition, this is a confirmation that indeed the polycaprolactone out of the ε-CL is formed and not another by product.

The acyltransferase activity of lipase CAL-A in a strictly aqueous phase was thus successfully demonstrated.

### Literature

Baldwin CVF, Wohlgemuth R, Woodley JM (2008) The first 200-L scale asymmetric Baeyer-Villiger oxidation using a whole-cell biocatalyst. Organic Process Research & Development 12: 660-665
Balke K, Kadow M, Mallin H, Sass S, Bornscheuer UT (2012) Discovery, application and protein engineering of Baeyer-Villiger monooxygenases for organic synthesis. Organic & biomolecular chemistry 10: 6249-6265
Bornscheuer UT, Kazlauskas RJ (2006a) Lipases and esterases: Sections 5.1 - 5.2. In Hydrolases in Organic Synthesis, pp 61-140. Wiley-VCH Verlag GmbH & Co. KGaA
Bornscheuer UT, Kazlauskas RJ (2006b) Lipases and esterases: Sections 5.3 - 5.4. In Hydrolases in Organic Synthesis, pp 141-183. Wiley-VCH Verlag GmbH & Co. KGaA
Bosley JA, Casey J, Macrae AR, MyCock G (1997) Process for the esterification of carboxylic acids with tertiary alcohols using a lipase from Candida antarctica. US Patent 5658769.
Braud C, Devarieux R, Atlan A, Ducos C, Vert M (1998) Capillary zone electrophoresis in normal or reverse polarity separation modes for the analysis of hydroxy acid oligomers in neutral phosphate buffer. Journal of Chromatography B 706: 73-82
Briand D, Dubreucq E, Galzy P (1994) Enzymatic fatty esters synthesis in aqueous-medium with lipase from Candida parapsilosis (Ashford) Langeron and Talice. Biotechnology Letters 16: 813-818
Briand D, Dubreucq E, Galzy P (1995a) Factors affecting the acyltransfer activity of the lipase from Candida parapsilosis in aqueous-media. Journal of the American Chemical Society 72: 1367-1373
Briand D, Dubreucq E, Galzy P (1995b) Functioning and regioselectivity of the lipase of Candida parapsilosis (Ashford) Langeron and Talice in aqueous-medium - New interpretation of regioselectivity taking acyl migration into account. European Journal of Biochemistry 228: 169-175
Briand D, Dubreucq E, Grimaud J, Galzy P (1995c) Substrate-specificity of the lipase from Candida parapsilosis. Lipids 30: 747-754
Casas J, Persson PV, Iversen T, Cordova A (2004) Direct organocatalytic ring-opening polymerizations of lactones. Advanced Synthesis & Catalysis 346: 1087-1089
Chandra R, Rustgi R (1998) Biodegradable polymers. Progress in Polymer Science 23: 1273-1335
Chen DR, Bei JZ, Wang SG (2000) Polycaprolactone microparticles and their biodegradation. Polymer Degradation and Stability 67: 455-459
Conrad HE, DuBus R, Gunsalus IC (1961) An enzyme system for cyclic ketone lactonization. Biochemical and Biophysical Research Communications 6: 293-297
de Gonzalo G, Mihovilovic MD, Fraaije MW (2010) Recent developments in the application of Baeyer-Villiger monooxygenases as biocatalysts. Chembiochem : a European journal of chemical biology 11: 2208-2231
De Kesel C, Wauven CV, David C (1997) Biodegradation of polycaprolactone and its blends with poly(vinylalcohol) by micro-organisms from a compost of house-hold refuse. Polymer Degradation and Stability 55: 107-113
Domínguez de María P, Carboni-Oerlemans C, Tuin B, Bargeman G, van der Meer A, van Gemert R (2005) Biotechnological applications of Candida antarctica lipase A: State-of-the-art. Journal of Molecular Catalysis B: Enzymatic 37: 36-46
Donoghue NA, Norris DB, Trudgill PW (1976) The Purification and properties of Cyclohexanone Oxygenase from Nocardia globerula CL1 and Acinetobacter NCIB 9871. European Journal of Biochemistry 63: 175-192
Ericsson DJ, Kasrayan A, Johanssonl P, Bergfors T, Sandstrom AG, Backvall JE, Mowbray SL (2008) X-ray structure of Candida antarctica lipase A shows a novel lid structure and a likely mode of interfacial activation. Journal of Molecular Biology 376: 109-119
Gedey S, Liljeblad A, Lázár L, Fülöp F, Kanerva LT (2001) Preparation of highly enantiopure β-amino esters by Candida antarctica lipase A. Tetrahedron: Asymmetry 12: 105-110
Geitner K, Rehdorf J, Snajdrova R, Bornscheuer UT (2010) Scale-up of Baeyer-Villiger monooxygenase-catalyzed synthesis of enantiopure compounds. Applied Microbiology And Biotechnology 88: 1087-1093
Goury V, Jhurry D, Bhaw-Luximon A, Novak BM, Belleney J (2005) Synthesis and characterization of random and block copolypeptides derived from gammamethylglutamate and leucine N-carboxyanhydrides. Biomacromolecules 6: 1987-1991
Gross RA, Kalra B (2002) Biodegradable polymers for the environment. Science 297: 803-807
Hedrick JL, Magbitang T, Connor EF, Glauser T, Volksen W, Hawker CJ, Lee VY, Miller RD (2002) Application of complex macromolecular architectures for advanced microelectronic materials. Chemistry - A European Journal 8: 3308-3319
Henke E, Bornscheuer UT (2002) Esterases from Bacillus subtilis and B. stearothermophilus share high sequence homology but differ substantially in their properties. Applied Microbiology and Biotechnology 60: 320-326
Henke E, Pleiss J, Bornscheuer UT (2002) Activity of lipases and esterases towards tertiary alcohols: insights into structure-function relationships. Angewandte Chemie, International Edition 41: 3211-3213
Hoegh I, Patkar S, Halkier T, Hansen MT (1995) Two lipases from Candida antarctica: cloning and expression in Aspergillus oryzae. Canadian Journal of Botany 73: 869-875
Hummel W (1997) New alcohol dehydrogenases for the synthesis of chiral compounds. New Enzymes for Organic Synthesis 58: 145-184.
Hutmacher DW, Schantz T, Zein I, Ng KW, Teoh SH, Tan KC (2001) Mechanical properties and cell cultural response of polycaprolactone scaffolds designed and fabricated via fused deposition modeling. Journal of Biomedical Materials Research Part B 55: 203-216
Ikada Y, Tsuji H (2000) Biodegradable polyesters for medical and ecological applications. Macromolecular Rapid Communications 21: 117-132
Inprakhon P, Panlawan P, Pongtharankul T, Marie E, Wiemann LO, Durand A, Sieber V (2014) Toward one-pot lipase-catalyzed synthesis of poly(epsilon-caprolactone) particles in aqueous dispersion. Colloids and Surfaces B: Biointerfaces 113: 254-60
Iroh JO (1999) Polymer Data Handbook, New York: Oxford University Press.
Jenkins MJ, Harrison KL, Silva MMCG, Whitaker MJ, Shakesheff KM, Howdle SM (2006) Characterisation of microcellular foams produced from semi-crystalline PCL using supercritical carbon dioxide. European Polymer Journal 42: 3145-3151
Joshi P, Madras G (2008) Degradation of polycaprolactone in supercritical fluids. Polymer Degradation and Stability 93: 1901-1908
Gröger H, Borchert S, Kraußer M, Hummel, W (2012) Enzyme-catalyzed asymmetric reduction of ketones. in: Drauz K, Gröger H, May O (eds.) Enzyme catalysis in Organic Synthesis, 3rd edition; Wiley-VCH Weinheim, 1037-1110
Kayser MM, Clouthier CM (2006) New bioorganic reagents: Evolved cyclohexanone monooxygenase - Why is it more selective? The Journal of Organic Chemistry 71: 8424-8430
Kobayashi S (1999) Enzymatic polymerization: A new method of polymer synthesis. Journal of Polymer Science Part A: Polymer Chemistry 37: 3041-3056
Kobayashi S, Kikuchi H, Uyama H (1997) Lipase-catalyzed ring-opening polymerization of 1,3-dioxan-2-one. Macromolecular Rapid Communications 18: 575-579
Kobayashi S, Takeya K, Suda S, Uyama H (1998a) Lipase-catalyzed ring-opening polymerization of medium-size lactones to polyesters. Macromolecular Chemistry and Physics 199: 1729-1736
Kobayashi S, Uyama H, Namekawa S (1998b) In vitro biosynthesis of polyesters with isolated enzymes in aqueous systems and organic solvents. Polymer Degradation and Stability 59: 195-201
Kobayashi S, Uyama H, Namekawa S, Hayakawa H (1998c) Enzymatic ring-opening polymerization and copolymerization of 8-octanolide by lipase catalyst. Macromolecules 31: 5655-5659
Kobayashi S, Uyama H, Ohmae M (2001) Enzymatic polymerization for precision polymer synthesis. Bulletin of the Chemical Society of Japan 74: 613-635
Kroutil W, Mang H, Edegger K, Faber K (2004) Biocatalytic oxidation of primary and secondary alcohols. Advanced Synthesis & Catalysis 346: 125-142
Labet M, Thielemans W (2009) Synthesis of polycaprolactone: a review. Chemical Society reviews 38: 3484-3504
Lam CXF, Teoh SH, Hutmacher DW (2007) Comparison of the degradation of polycaprolactone and polycaprolactone-(beta-tricalcium phosphate) scaffolds in alkaline medium. Polymer International 56: 718-728
Leipold, F., Wardenga, R., Bornscheuer, U.T. (2012), Cloning, expression and characterisation of an eukaryotic cycloalkanone monooxygenase from Cylindrocarpon radicicola ATCC 11011, Appl. Microb. Biotechno/., 94, 705-717
Liljeblad A, Kanerva LT (2006) Biocatalysis as a profound tool in the preparation of highly enantiopure β-amino acids. Tetrahedron 62: 5831-5854
Mallin H, Wulf H, Bornscheuer UT (2013) A self-sufficient Baeyer-Villiger biocatalysis system for the synthesis of ε-caprolactone from cyclohexanol. Enzyme and microbial technology 53: 283-287
Matsumura S, Beppu H, Tsukada K, Toshima K (1996) Enzyme-catalyzed ring-opening polymerization of beta-propiolactone. Biotechnology Letters 18: 1041-1046
Matsumura S, Mabuchi K, Toshima K (1997) Lipase-catalyzed ring-opening polymerization of lactide. Macromolecular Rapid Communications 18: 477-482
Muller J, Fredrich B, Kohlmann C, Maksym L, Bornscheuer UT (2013), A high-throughput assay for the determination of acyltransferase activity of lipase CAL-A. European Journal of Lipid Science and Technology doi: 10.1002/ejlt.201300226
Müller, J., Fredrich, B., Kohlmann, C., Maksym, L., Bornscheuer, U.T. (2014), A high-throughput assay for the determination of acyltransferase activity of lipase CAL-A, Eur. J. Lipid Sci. Technol.*,* doi/10.1002/ejlt.201300226
Namekawa S, Uyama, H, Kobayashi, S (1998) Lipase-catalyzed ring-opening polymerization of lactones in water. Polymer Journal 30: 269-271.
Neugnot V, Moulin G, Dubreucq E, Bigey F (2002) The lipase/acyltransferase from Candida parapsilosis - Molecular cloning and characterization of purified recombinant enzymes. European Journal of Biochemistry 269: 1734-1745
Nobes GAR, Kazlauskas RJ, Marchessault RH (1996) Lipase-catalyzed ring-opening polymerization of lactones: A novel route to poly(hydroxyalkanoate)s. Macromolecules 29: 4829-4833
Panlawan P, Luangthongkam P, Wiemann LO, Sieber V, Marie E, Durand A, Inprakhon P (2013) Lipase-catalyzed interfacial polymerization of omega-pentadecalactone in aqueous biphasis medium: a mechanistic study, Journal of Molecular Catalysis B: Enzymatic 88: 69-76
Panova AA, Taktak S, Randriamahefa S, Cammas-Marion S, Guerin P, Kaplan DL (2003) Polymerization of propyl malolactonate in the presence of Candida rugosa lipase. Biomacromolecules 4: 19-27
Pena J, Corrales T, Izquierdo-Barba I, Doadrio AL, Vallet-Regi M (2006) Long term degradation of poly(epsilon-caprolactone) films in biologically related fluids. Polymer Degradadation and Stability 91: 1424-1432
Persson PV, Schroder J, Wickholm K, Hedenstrom E, Iversen T (2004) Selective organocatalytic ring-opening polymerization: A versatile route to carbohydrate-functionalized poly(is an element of-caprolactones). Macromolecules 37: 5889-5893
Reid MF, Fewson CA (1994) Molecular characterization of microbial alcohol dehydrogenases. Critical Reviews in Microbiology 20: 13-56
MacDonald RT, Pulapura SK, Svirkin YY, Gross RA (1995) Enzyme-catalyzed ε-caprolactone ring-opening polymerisation. Macromolecules 28: 73 - 78
Riaublanc A, Ratomahenina R, Galzy P, Nicolas M (1993) Peculiar properties of lipase from Candida parapsilosis (Ashford) Langeron and Talice. Journal of the American Oil Chemists' Society 70: 497-500
Rocca MC, Carr G, Lambert AB, MacQuarrie DJ, Clark JH (2003) Process for the oxidation of cyclohexanone to ε-caprolactone. US Patent 6531615 B2.
Schmidt M, Henke E, Heinze B, Kourist R, Hidalgo A, Bornscheuer UT (2007) A versatile esterase from Bacillus subtilis: Cloning, expression, characterization, and its application in biocatalysis. Biotechnol Journal 2: 249-253
Sheldon RA, "Organic synthesis; past, present and future", Chem. Ind.(London), 1992, 903-906
Sinha VR, Bansal K, Kaushik R, Kumria R, Trehan A (2004a) Poly-epsilon-caprolactone microspheres and nanospheres: an overview. International Journal of Pharmaceutics 278: 1-23
Sinha VR, Singla AK, Wadhawan S, Kaushik R, Kumria R, Bansal K, Dhawan S (2004b) Chitosan microspheres as a potential carrier for drugs. International Journal of Pharmaceutics 274: 1-33
Staudt S, Bornscheuer UT, Menyes U, Hummel W, Gröger H (2013a) Direct biocatalytic one-pot-transformation of cyclohexanol with molecular oxygen into ε-caprolactone. Enzyme and microbial technology 53: 288-292
Staudt S, Burda E, Giese C, Muller CA, Marienhagen J, Schwaneberg U, Hummel W, Drauz K, Groger H (2013) Direct oxidation of cycloalkanes to cycloalkanones with oxygen in Water. Angewandte Chemie, International Edition 52: 2359-2363
Stewart JD (1998) Cyclohexanone monooxygenase: A useful reagent for asymmetric Baeyer-Villiger reactions. Current Organic Chemistry 2: 195-216
Uyama H, Takeya K, Kobayashi S (1995) Enzymatic ring-opening polymerization of lactones to polyesters by lipase catalyst: Unusually high reactivity of macrolides. Bulletin of the Chemical Society of Japan 68: 56-61
Verger R, Schmid RD (1998) Lipases: Interfacial enzymes with attractive applications. Angewandte Chemie, International Edition 37: 1606-1633
Weckbecker A, Hummel W (2006) Cloning, expression, and characterization of an (R)-specific alcohol dehydrogenase from Lactobacillus kefir. Biocatalysis and Biotransformation 24: 380-389

## Claims

1. Method for producing polyesters, wherein a lactone is converted to a polyester by a lipase, **characterized**
- **in that** the lipase is Lipase A from *Candida antarctica* (CAL-A) according to SEQ ID NO 1 or a homologue enzyme having sequence identity of at least 25% with SEQ ID NO 1 and having the same function as CAL-A
and
- **in that** the conversion is done in an essentially aqueous medium.

2. Method for producing polyesters according to claim 1, **characterized in that** the lactone is selected from formula I, 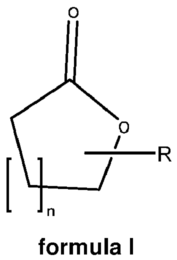 wherein the lactone has at least one substituent R at variable position of the lactone ring,
R being selected from hydrogen or C1-C20-alkyl, -alkenyl, -alkinyl or C1-C20-cycloalkyl, -cycloalkenyl, -cycloalkinyl or C1-C20-aryl, wherein in case of more than one aliphatic or aromatic ring system the ring systems are condensed or separate and
n being selected from 0-17.

3. Method for producing polyesters from cycloalkanones, characterized that in one vessel and in an essentially aqueous medium
a cycloalkanone is converted with
- a Baeyer-Villiger-Monooxygenase (BVMO) and
- Lipase A from *Candida antarctica* (CAL-A) according to SEQ ID NO 1 or a homologue enzyme having sequence identity of at least 25% with SEQ ID NO 1 and having the same function as CAL-A,
which convert the cycloalkanone into the polyester.

4. Method for producing polyesters from cycloalkanones according to claim 3,
wherein the cycloalkanone is selected from formula II, 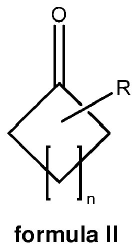 wherein the cycloalkanone has at least one substituent R at variable ring position,
R being selected from hydrogen or C1-C20-alkyl, -alkenyl, -alkinyl or C1-C20-cycloalkyl, -cycloalkenyl, -cycloalkinyl or C1-C20-aryl, wherein in case of more than one aliphatic or aromatic ring system the ring systems are condensed or separate and
n being selected from 0-17.

5. Method for producing polyesters from cycloalkanols, characterized that in one vessel and in an essentially aqueous medium
a cycloalkanol is converted with
- an alcohol dehydrogenase (ADH),
- a Baeyer-Villiger-Monooxygenase (BVMO) and
- Lipase A from *Candida antarctica* (CAL-A) according to SEQ ID NO 1 or a homologue enzyme having sequence identity of at least 25% with SEQ ID NO 1 and having the same function as CAL-A,
which convert the cycloalkanol into the polyester.

6. Method for producing polyesters from cycloalkanols according to claim 5, wherein the cycloalkanol is selected from formula III, 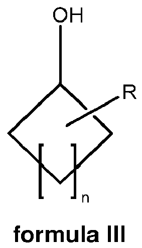 wherein the cycloalkanol has at least one substituent R at variable ring position,
R being selected from hydrogen or C1-C20-alkyl, alkenyl, -alkinyl or C1-C20-cycloalkyl, -cycloalkenyl, -cycloalkinyl or C1-C20-aryl, wherein in case of more than one aliphatic or aromatic ring system the ring systems are condensed or separate and
n being selected from 0-17.

7. Method for producing polyesters according to anyone of claims 2, 4 or 6, **characterized in that**
R is selected from C1-C6-alkyl, more preferably linear unbranched C1-C4-alkyl, most preferred methyl.

8. Method for producing polyesters according to anyone of claims 2, 4 or 6, **characterized in that**
n is selected from 1-9, more preferred from 1-4, most preferred 3
or
n is selected from 10-16, more preferred from 12-15, most preferred 12.

9. Method for producing polyesters according to anyone of claims 1-8, wherein the lactone of formula I or the cycloalkanone of formula II or the cycloalkanol of formula III has at least one C-atom of the cyclic structure replaced by a hetero atom, preferably selected from the 15^{th} or 16^{th} group of the periodic table, more preferably selected from nitrogen, oxygen or sulphur, most preferred nitrogen.

10. Method for producing polyesters according to claim 9, wherein the at least one hetero atom, especially the at least one nitrogen atom, has a substituent R1, which is selected from hydrogen or C1-C20-alkyl, alkenyl, -alkinyl or C1-C20-cycloalkyl, -cycloalkenyl, -cycloalkinyl or C1-C20-aryl, wherein in case of more than one aliphatic or aromatic ring system the ring systems are condensed or separated.

11. Method for producing polyesters according to anyone of claims 1-10, wherein the homologue enzyme has sequence identity of at least 50%, more preferred of at least 65%, more preferred of at least 90%, most preferred of at least 95% with SEQ ID NO 1 and has the same function as CAL-A.

12. Method for producing polyesters according to anyone of claims 1-11, wherein the homologue enzyme is selected from
Lipase from *Pseudozyma antarctica* (CAL-A_pseu., SEQ ID NO 2),
Lipase from *Kurtzmanomyces* sp. I-11 (Lip_K, SEQ ID NO 3),
Lipase from *Pseudozyma hubeiensis* (Lip_PseuHub, SEQ ID NO 4),
Lipase from *Ustilago hordei* (Lip_UstHor, SEQ ID NO 5),
Lipase from *Ustilago maydis* (Lip_UM03410, SEQ ID NO 6),
Lipase from *Pseudozyma* sp. GHG001 (Lip_PSEUBRA-1g, SEQ ID NO 7),
Lipase from *Sporisorium reilianum* (Lip_SpoRei_E7, SEQ ID NO 8),
Lipase from *Sporisorium reilianum* (Lip_SpoRei_E6, SEQ ID NO 9),
Lipase from *Pseudozyma* sp. GHG001 (Lip_PSEUBRA-1g, SEQ ID NO 10),
Lipase from *Candida parapsilosis* (CpLIP 2, SEQ ID NO 11).

13. Method for producing polyesters according to anyone of claims 1-12, wherein the homologue enzyme is selected from
Lipase from *Pseudozyma antarctica* (CAL-A_pseu., SEQ ID NO 2),
Lipase from *Ustilago maydis* (Lip_UM03410, SEQ ID NO 6),
Lipase from *Candida parapsilosis* (CpLIP 2, SEQ ID NO 11).

14. Method for producing polyesters according to anyone of claims 1-13, wherein the homologue enzyme is lipase from *Pseudozyma antarctica* (CAL-A_pseu., SEQ ID NO 2).


<110>  Ernst-Moritz-Arndt-Universität Greifswald
       Enzymicals AG

<120>  Process for the enzymatic production of oligo-/polyesters

<130>  PVA-25 895 EP

<160>  13

<170>  PatentIn version 3.5

<210>  1
<211>  462
<212>  PRT
<213>  Candida antarctica

<400>  1

Met Arg Val Ser Leu Arg Ser Ile Thr Ser Leu Leu Ala Ala Ala Thr
1               5                   10                  15

Ala Ala Val Leu Ala Ala Pro Ala Ala Glu Thr Leu Asp Arg Arg Ala
            20                  25                  30

Ala Leu Pro Asn Pro Tyr Asp Asp Pro Phe Tyr Thr Thr Pro Ser Asn
        35                  40                  45

Ile Gly Thr Phe Ala Lys Gly Gln Val Ile Gln Ser Arg Lys Val Pro
    50                  55                  60

Thr Asp Ile Gly Asn Ala Asn Asn Ala Ala Ser Phe Gln Leu Gln Tyr
65                  70                  75                  80

Arg Thr Thr Asn Thr Gln Asn Glu Ala Val Ala Asp Val Ala Thr Val
                85                  90                  95

Trp Ile Pro Ala Lys Pro Ala Ser Pro Pro Lys Ile Phe Ser Tyr Gln
            100                 105                 110

Val Tyr Glu Asp Ala Thr Ala Leu Asp Cys Ala Pro Ser Tyr Ser Tyr
        115                 120                 125

Leu Thr Gly Leu Asp Gln Pro Asn Lys Val Thr Ala Val Leu Asp Thr
    130                 135                 140

Pro Ile Ile Ile Gly Trp Ala Leu Gln Gln Gly Tyr Tyr Val Val Ser
145                 150                 155                 160

Ser Asp His Glu Gly Phe Lys Ala Ala Phe Ile Ala Gly Tyr Glu Glu
                165                 170                 175

Gly Met Ala Ile Leu Asp Gly Ile Arg Ala Leu Lys Asn Tyr Gln Asn

180                 185                 190

Leu Pro Ser Asp Ser Lys Val Ala Leu Glu Gly Tyr Ser Gly Gly Ala
        195                 200                 205

His Ala Thr Val Trp Ala Thr Ser Leu Ala Glu Ser Tyr Ala Pro Glu
    210                 215                 220

Leu Asn Ile Val Gly Ala Ser His Gly Gly Thr Pro Val Ser Ala Lys
225                 230                 235                 240

Asp Thr Phe Thr Phe Leu Asn Gly Gly Pro Phe Ala Gly Phe Ala Leu
                245                 250                 255

Ala Gly Val Ser Gly Leu Ser Leu Ala His Pro Asp Met Glu Ser Phe
            260                 265                 270

Ile Glu Ala Arg Leu Asn Ala Lys Gly Gln Arg Thr Leu Lys Gln Ile
        275                 280                 285

Arg Gly Arg Gly Phe Cys Leu Pro Gln Val Val Leu Thr Tyr Pro Phe
    290                 295                 300

Leu Asn Val Phe Ser Leu Val Asn Asp Thr Asn Leu Leu Asn Glu Ala
305                 310                 315                 320

Pro Ile Ala Ser Ile Leu Lys Gln Glu Thr Val Val Gln Ala Glu Ala
                325                 330                 335

Ser Tyr Thr Val Ser Val Pro Lys Phe Pro Arg Phe Ile Trp His Ala
            340                 345                 350

Ile Pro Asp Glu Ile Val Pro Tyr Gln Pro Ala Ala Thr Tyr Val Lys
        355                 360                 365

Glu Gln Cys Ala Lys Gly Ala Asn Ile Asn Phe Ser Pro Tyr Pro Ile
    370                 375                 380

Ala Glu His Leu Thr Ala Glu Ile Phe Gly Leu Val Pro Ser Leu Trp
385                 390                 395                 400

Phe Ile Lys Gln Ala Phe Asp Gly Thr Thr Pro Lys Val Ile Cys Gly
                405                 410                 415

Thr Pro Ile Pro Ala Ile Ala Gly Ile Thr Thr Pro Ser Ala Asp Gln
            420                 425                 430

Val Leu Gly Ser Asp Leu Ala Asn Gln Leu Arg Ser Leu Asp Gly Lys
        435                 440                 445

Gln Ser Ala Phe Gly Lys Pro Phe Gly Pro Ile Thr Pro Pro
    450                 455                 460

<210>  2
<211>  462
<212>  PRT
<213>  Pseudozyma antarctica

<400>  2

Met Arg Val Ser Leu Arg Ser Ile Thr Ser Leu Leu Ala Ala Ala Thr
1               5                   10                  15

Ala Ala Val Leu Ala Ala Pro Ala Ala Glu Thr Leu Asp Arg Arg Ala
            20                  25                  30

Ala Leu Pro Asn Pro Tyr Asp Asp Pro Phe Tyr Thr Thr Pro Ser Asn
        35                  40                  45

Ile Gly Thr Phe Ala Lys Gly Gln Val Ile Gln Ser Arg Lys Val Pro
    50                  55                  60

Thr Asp Ile Gly Asn Ala Asn Asn Ala Ala Ser Phe Gln Leu Gln Tyr
65                  70                  75                  80

Arg Thr Thr Asn Thr Gln Asn Glu Ala Val Ala Asp Val Ala Thr Val
                85                  90                  95

Trp Ile Pro Ala Lys Pro Ala Ser Pro Pro Lys Ile Phe Ser Tyr Gln
            100                 105                 110

Val Tyr Glu Asp Ala Thr Ala Leu Asp Cys Ala Pro Ser Tyr Ser Tyr
        115                 120                 125

Leu Thr Gly Leu Asp Gln Pro Asn Lys Val Thr Ala Val Leu Asp Thr
    130                 135                 140

Pro Ile Ile Ile Gly Trp Ala Leu Gln Gln Gly Tyr Tyr Val Val Ser
145                 150                 155                 160

Ser Asp His Glu Gly Phe Lys Ala Ala Phe Ile Ala Gly Tyr Glu Glu
                165                 170                 175

Gly Met Ala Ile Leu Asp Gly Ile Arg Ala Leu Lys Asn Tyr Gln Asn
            180                 185                 190

Leu Pro Ser Asp Ser Lys Val Ala Leu Glu Gly Tyr Ser Gly Gly Ala
        195                 200                 205

His Ala Thr Val Trp Ala Thr Ser Leu Ala Glu Ser Tyr Ala Pro Glu
    210                 215                 220

Leu Asn Ile Val Gly Ala Ser His Gly Gly Thr Pro Val Ser Ala Lys
225                 230                 235                 240

Asp Thr Phe Thr Phe Leu Asn Gly Gly Pro Phe Ala Gly Phe Ala Leu
                245                 250                 255

Ala Gly Val Ser Gly Leu Ser Leu Ala His Pro Asp Met Glu Ser Phe
            260                 265                 270

Ile Glu Ala Arg Leu Asn Ala Lys Gly Gln Gln Ala Leu Lys Gln Ile
        275                 280                 285

Arg Gly Arg Gly Phe Cys Leu Pro Gly Val Val Leu Thr Tyr Pro Phe
    290                 295                 300

Leu Asn Val Phe Ser Leu Val Asn Asp Thr Asn Leu Leu Asn Glu Ala
305                 310                 315                 320

Pro Ile Val Ser Ile Leu Lys Gln Glu Thr Val Val Gln Ala Glu Ala
                325                 330                 335

Ser Tyr Thr Val Ser Val Pro Lys Phe Pro Arg Phe Ile Trp His Ala
            340                 345                 350

Val Pro Asp Glu Ile Val Pro Tyr Gln Pro Ala Ala Thr Tyr Val Lys
        355                 360                 365

Glu Gln Cys Ala Lys Gly Ala Asn Ile Asn Phe Ser Pro Tyr Pro Ile
    370                 375                 380

Ala Glu His Leu Thr Ala Glu Ile Phe Gly Leu Val Pro Ser Leu Trp
385                 390                 395                 400

Phe Ile Lys Gln Ala Phe Asp Gly Thr Thr Pro Lys Val Ile Cys Gly
                405                 410                 415

Thr Pro Ile Pro Ala Ile Ala Gly Ile Thr Thr Pro Ser Ala Asp Gln
            420                 425                 430

Val Leu Gly Ser Asp Leu Ala Asp Gln Leu Arg Ser Leu Asn Gly Lys
        435                 440                 445

Gln Ser Ala Phe Gly Lys Pro Phe Gly Pro Ile Thr Pro Pro
    450                 455                 460

<210>  3

<211>  458
<212>  PRT
<213>  Kurtzmanomyces sp.

<400>  3

Met Arg Phe Phe Leu Arg Ala Val Leu Gly Leu Ala Val Thr Ala Thr
1               5                   10                  15

Ala Ala Leu Ala Ala Pro Leu Glu Pro Arg Ala Ala Leu Pro Asp Pro
            20                  25                  30

Asn Glu Asp Pro Phe Tyr Ser Thr Pro Ser Asn Ile Glu Thr Phe Ala
        35                  40                  45

Asn Gly Gln Ile Ile Gln Ser Arg Lys Val Pro Thr Asp Ile Gly Asn
    50                  55                  60

Ser Asn Asn Ala Ala Ser Tyr Gln Leu Ser Tyr Arg Thr Thr Asn Thr
65                  70                  75                  80

Gln Glu Asp Ala Val Ala Asn Val Ala Thr Ile Trp Ile Pro Ala Lys
                85                  90                  95

Pro Ser Ser Pro Pro Arg Ile Phe Thr Tyr Gln Val Tyr Glu Asp Ser
            100                 105                 110

Thr Gln Leu Asp Cys Ala Pro Ser Tyr Ser Tyr Leu Thr Gly Tyr Asp
        115                 120                 125

Gln Pro Asn Lys Ala Thr Ala Val Leu Asp Thr Pro Ile Val Ile Ser
    130                 135                 140

Trp Ala Leu Gln Gln Gly Tyr Tyr Val Val Ser Ala Asp His Glu Gly
145                 150                 155                 160

Ala Arg Ser Ala Phe Ile Ala Gly Tyr Glu Glu Gly Met Ala Ala Leu
                165                 170                 175

Asp Gly Ile Arg Ala Leu Arg Asn Tyr Ala Lys Leu Pro Gln Asp Ser
            180                 185                 190

Ala Val Gly Ala Tyr Gly Tyr Ser Gly Gly Ala His Ala Thr Val Trp
        195                 200                 205

Ala Thr Ser Leu Ala Ala Ala Tyr Ala Pro Glu Ile Asn Phe Ile Gly
    210                 215                 220

Ala Ala His Gly Gly Thr Pro Val Ser Ala Lys Asp Thr Phe Thr Phe
225                 230                 235                 240

Ile Asn Gly Gly Phe Phe Ala Gly Phe Ala Ile Ala Gly Val Ser Gly
                245                 250                 255

Leu Ala Asn Ala His Pro Asp Met Glu Ala Phe Ile Gln Pro Arg Leu
            260                 265                 270

Asn Ala Glu Gly Val Lys Thr Leu Lys Gln Ile Arg Ser Arg Gly Phe
        275                 280                 285

Cys Leu Pro Glu Val Val Thr Thr Tyr Pro Phe Lys Asn Val Phe Ala
    290                 295                 300

Leu Val Asn Asp Thr Asn Leu Leu Thr Glu Gln Pro Ile Ser Gly Ile
305                 310                 315                 320

Leu Gln Gln Glu Thr Leu Val Gln Ser Glu Ala Ser Tyr Ala Val Pro
                325                 330                 335

Val Pro Lys Phe Pro Arg Phe Leu Trp His Ala Ala Leu Asp Glu Ile
            340                 345                 350

Val Pro Tyr Val Pro Val Thr Glu Tyr Val Lys Glu Gln Cys Ala Lys
        355                 360                 365

Gly Ala Asn Ile Asn Phe Asn Thr Tyr Pro Ile Ala Glu His Leu Thr
    370                 375                 380

Ala Glu Ile Phe Gly Leu Val Pro Gly Leu Trp Phe Leu Ser Gln Ala
385                 390                 395                 400

Tyr Glu Gly Lys Ala Pro Ala Val Gln Cys Gly Thr Ala Leu Pro Ala
                405                 410                 415

Ala Pro Ser Ala Gln Gln Val Leu Gly Asn Asp Leu Ala Asn Gln Leu
            420                 425                 430

Ser Ser Leu Asn Gly Lys Gln Ser Pro Phe Gly Lys Pro Phe Gly Pro
        435                 440                 445

Ile Ser Pro Thr Ser Leu Asp Lys Leu Leu
    450                 455

<210>  4
<211>  430
<212>  PRT
<213>  Pseudozyma hubeiensis

<400>  4

Met His Thr Arg Glu Thr Leu Pro Asp Pro Asn Asp Asp Pro Phe Tyr

1               5                   10                  15

Ala Gln Pro Ser Asn Ile Ala Ser Tyr Ala Asn Gly Gln Val Ile Gln
            20                  25                  30

Ser Arg Lys Val Asp Thr Glu Ile Gly Ser Ile Asn Asn Val Asn Thr
        35                  40                  45

Phe Gln Val Lys Tyr Arg Thr Thr Asn Thr Gln Asn Gln Ala Gln Ser
    50                  55                  60

Asn Leu Ala Thr Val Trp Ile Pro Ser Lys Pro Ala Ser Pro Pro Lys
65                  70                  75                  80

Ile Phe Ser Tyr Gln Val Tyr Met Asp Ala Thr Gln Ile Asp Cys Ala
                85                  90                  95

Pro Ser Tyr Ser Tyr Val Thr Gly Leu Asp Lys Pro Gly Lys Gly Thr
            100                 105                 110

Val Ile Leu Asp Thr Pro Ile Val Ile Ser Trp Ala Leu Gln Gln Gly
        115                 120                 125

Tyr Tyr Val Val Ser Ser Asp Asp Glu Gly Pro Arg Ala Ala Phe Ile
    130                 135                 140

Ala Gly Tyr Glu Glu Gly Lys Ala Val Leu Asp Gly Ile Arg Ala Leu
145                 150                 155                 160

Gln Asn Tyr Ala Asn Leu Pro Lys Asp Ser Pro Ile Gly Met Tyr Gly
                165                 170                 175

Tyr Ser Gly Gly Ala His Thr Thr Gly Trp Ala Val Asn Leu Ala Gly
            180                 185                 190

Ser Tyr Ala Ser Glu Leu Asn Ile Ile Gly Ala Ala Tyr Gly Gly Leu
        195                 200                 205

Pro Ala Ser Ala Lys Asp Thr Phe Thr Phe Leu Asn Gly Lys Ser Ile
    210                 215                 220

Phe Ser Gly Phe Ala Leu Ala Gly Val Ser Gly Leu Ala Leu Gly His
225                 230                 235                 240

Pro Asp Met Glu Ala Phe Ile Lys Pro Arg Leu Asn Ala Lys Gly Asn
                245                 250                 255

Gln Thr Leu Ala Gln Ile Arg Ser Arg Gly Glu Cys Ile Gly Gln Val
            260                 265                 270

Ala Leu Gly Tyr Pro Phe Leu Asp Val Phe Ser Leu Val Asn Asp Thr
        275                 280                 285

Asn Leu Leu Asn Glu Glu Pro Ile Val Ser Ile Leu Lys Thr Glu Thr
    290                 295                 300

Leu Val Gln Ala Glu Ala Ser Tyr Thr Val Pro Val Pro Lys Phe Pro
305                 310                 315                 320

Arg Phe Met Trp His Ala Leu Pro Asp Glu Ile Val Pro Phe Gln Pro
                325                 330                 335

Ala Gln Asp Tyr Val Thr Glu Gln Cys Ala Lys Gly Ala Asp Ile Asn
            340                 345                 350

Trp Asn Val Tyr Pro Ile Ala Glu His Ile Ser Ala Glu Leu Leu Gly
        355                 360                 365

Leu Leu Pro Gly Ile Asp Trp Leu Ser Lys Ala Tyr Lys Gly Gln Ala
    370                 375                 380

Pro Lys Val Ala Cys Gly Gly Gly Val Pro Ala Thr Pro Pro Pro Ala
385                 390                 395                 400

Glu Asp Val Leu Gly Gly Asp Leu Ala Ser Gln Phe Arg Ser Leu Asn
                405                 410                 415

Gly Lys Gln Ser Ala Tyr Gly Lys Pro Phe Asn Ala Gly Phe
            420                 425                 430

<210>  5
<211>  458
<212>  PRT
<213>  Ustilago hordei

<400>  5

Met Arg Phe Phe Leu Gly Ala Val Leu Ala Leu Ala Ala Ala Ala Ile
1               5                   10                  15

Ala Ala Leu Ala Val Pro Met Glu Arg Arg Ala Gln Phe Pro Asp Pro
            20                  25                  30

Asn Glu Asp Pro Phe Tyr Gln Gln Pro Ser Asn Val Ala Thr Tyr Ala
        35                  40                  45

Asn Gly Gln Ile Ile Arg Ser Arg Lys Ala Asp Thr Asp Ile Gly Asn
    50                  55                  60

Ser Asn Lys Ala Gln Ala Phe Gln Leu Ser Tyr Arg Thr Thr Asn Thr
65                  70                  75                  80

Gln Gln Gln Ala Gln Ala Asn Val Ala Thr Val Trp Ile Pro Ala Lys
                85                  90                  95

Pro Ala Ser Pro Pro Lys Ile Phe Ser Tyr Gln Val Tyr Glu Asp Ser
            100                 105                 110

Thr Gln Leu Asp Cys Ala Pro Ser Tyr Ser Tyr Leu Ala Gly Phe Asp
        115                 120                 125

Pro Pro Asn Lys Ala Thr Thr Ile Leu Asp Thr Ser Leu Ile Ile Gly
    130                 135                 140

Trp Ala Leu Gln Gln Gly Tyr Tyr Val Leu Ser Ser Asp His Glu Gly
145                 150                 155                 160

Pro Arg Ala Ala Phe Ile Ala Gly Tyr Glu Glu Gly Met Ala Ile Leu
                165                 170                 175

Asp Ala Ile Arg Ala Leu Gln Asn Phe Arg His Leu Pro Lys Asp Ser
            180                 185                 190

Pro Val Gly Met Tyr Gly Tyr Ser Gly Gly Ala His Ala Thr Val Trp
        195                 200                 205

Ala Glu Ser Leu Ala Gly Ser Tyr Ala Pro Glu Ile Asn Ile Ile Ala
    210                 215                 220

Thr Ala His Gly Gly Thr Pro Phe Ser Thr Lys Asp Thr Phe Thr Phe
225                 230                 235                 240

Ile Asn Gly Gly Val Phe Ala Gly Phe Ala Ile Ala Gly Val Ser Gly
                245                 250                 255

Leu Ala Leu Val His Pro Ala Met Gln Ala Tyr Ile Lys Pro Arg Leu
            260                 265                 270

Asn Ala Gln Gly Val Lys Val Phe Gln Gln Ile Arg Ser Arg Ala Phe
        275                 280                 285

Cys Ile Ala Gln Val Val Phe Thr Tyr Pro Phe Thr Asn Val Phe Asn
    290                 295                 300

Leu Val Asn Gly Thr Asp Leu Leu Asn Gln Glu Pro Ile Arg Ser Ile
305                 310                 315                 320

Leu Lys Arg Glu Thr Leu Val Gln Ser Glu Ala Ser Cys Asp Val Pro

325                 330                 335

Val Val Arg Ala Pro Arg Phe Ile Trp His Ala Ala Leu Asp Glu Ile
            340                 345                 350

Val Pro Tyr Ala Pro Ala Ala Gln Tyr Val Lys Glu Gln Cys Asp Lys
        355                 360                 365

Gly Ala Gln Ile His Phe Glu Thr Tyr Pro Ile Ala Glu His Phe Ser
    370                 375                 380

Ala Glu Phe Phe Gly Leu Val Pro Ala Leu Trp Phe Leu Ser Gln Ala
385                 390                 395                 400

Tyr Ala Gly Lys Ala Ala Lys Thr Val Cys Gly Thr Ser Ile Pro Ala
                405                 410                 415

Ile Pro Gly Phe Thr Val Pro Ser Ala Glu Glu Val Leu Gly Ala Asp
            420                 425                 430

Leu Ala Lys Gln Leu Lys Gly Leu Ser Ala Lys Asp Leu Ser Ala Lys
        435                 440                 445

Asp Leu Ser Gly Lys His Leu Pro Ala Leu
    450                 455

<210>  6
<211>  582
<212>  PRT
<213>  Ustilago maydis

<400>  6

Met Trp Gly Arg Ile Arg Asn Val Ile Gln Pro Thr Trp Ala Pro Pro
1               5                   10                  15

Leu Phe Gly Thr Leu Asn Ile Ile Phe Ser Leu Phe Phe Arg Ala Gly
            20                  25                  30

Ile Ala Arg Ser His Lys Trp Thr Trp Cys Cys Tyr Arg Pro Thr Arg
        35                  40                  45

Met Ala Arg Ser Arg Thr Phe Ser Asn Ser Ala Pro Thr Arg Arg Arg
    50                  55                  60

Pro Glu Arg Leu Arg Leu Gln Lys Gly Ser Ser Asn Thr Thr Ile Arg
65                  70                  75                  80

Pro Arg Pro Ser Ala Ile Leu Pro Asp Glu Met Asn His Gly Ser Leu
                85                  90                  95

Leu Thr Val Val Pro His Thr Val Val Ala Ser Thr Pro Ser Phe Arg
            100                 105                 110

Ser Ser Phe Pro Asp Ser Leu Ile Ala Ser Val Gln Met Arg Phe Ile
        115                 120                 125

Ala Val Arg Ala Ile Val Thr Leu Ala Ala Ala Ala Ala Val Ser Leu
    130                 135                 140

Ala Val Pro Thr Glu Arg Arg Ala Ala Phe Ala Asp Pro Asn Asp Asp
145                 150                 155                 160

Leu Phe Tyr Thr Thr Pro Asp Asn Ile Asn Thr Tyr Ala Asn Gly Gln
                165                 170                 175

Val Ile Gln Ser Arg Lys Ala Asp Thr Asp Ile Gly Asn Ser Asn Lys
            180                 185                 190

Val Glu Ala Phe Gln Leu Gln Tyr Arg Thr Thr Asn Thr Gln Lys Glu
        195                 200                 205

Ala Gln Ala Asn Val Ala Thr Val Trp Ile Pro Asn Lys Pro Ala Ser
    210                 215                 220

Pro Pro Lys Ile Phe Ser Tyr Gln Val Tyr Gln Asp Ser Thr Gln Leu
225                 230                 235                 240

Asn Cys Ala Pro Ser Tyr Ser Phe Leu Lys Gly Leu Asp Lys Pro Asn
                245                 250                 255

Lys Ala Thr Thr Ile Leu Glu Ala Pro Ile Ile Ile Gly Trp Ala Leu
            260                 265                 270

Gln Gln Gly Phe Tyr Val Val Ser Ser Asp His Glu Gly Pro Arg Ser
        275                 280                 285

Ser Phe Ile Ala Gly Tyr Glu Glu Gly Met Ala Ile Leu Asp Gly Ile
    290                 295                 300

Arg Ala Leu Lys Asn Tyr Ala Lys Leu Pro Thr Asp Ser Ala Ile Gly
305                 310                 315                 320

Phe Tyr Gly Tyr Ser Gly Gly Ala His Ala Thr Gly Trp Ala Ala Asn
                325                 330                 335

Leu Ala Gly Ser Tyr Ala Pro Glu His Asn Ile Ile Gly Ala Ala Tyr
            340                 345                 350

Gly Gly Leu Pro Ala Ser Ala Arg Asp Thr Phe Asn Phe Leu Asn Lys
        355                 360                 365

Gly Ala Phe Ala Gly Phe Ala Ile Ala Gly Val Ser Gly Leu Ala Leu
    370                 375                 380

Ala Tyr Pro Asp Val Glu Thr Tyr Ile Gln Ser Arg Leu Asn Ala Lys
385                 390                 395                 400

Gly Glu Lys Val Phe Lys Gln Val Arg Ser Arg Gly Phe Cys Ile Gly
                405                 410                 415

Gln Val Val Leu Thr Tyr Pro Phe Val Asp Ala Tyr Ser Leu Ile Asn
            420                 425                 430

Asp Thr Asn Leu Leu Asn Glu Glu Pro Val Ala Ser Thr Leu Lys Ser
        435                 440                 445

Glu Thr Leu Val Gln Ala Glu Ala Ser Tyr Thr Val Pro Val Pro Lys
    450                 455                 460

Phe Pro Arg Phe Ile Trp His Ala Leu Leu Asp Glu Ile Val Pro Phe
465                 470                 475                 480

His Ser Ala Ala Thr Tyr Val Lys Glu Gln Cys Ser Lys Gly Ala Asp
                485                 490                 495

Ile Asn Trp Asn Val Tyr Ser Phe Ala Glu His Ile Ser Ala Glu Leu
            500                 505                 510

Phe Gly Leu Leu Pro Gly Leu Asp Trp Leu Asn Lys Ala Tyr Lys Gly
        515                 520                 525

Gln Ala Pro Lys Val Pro Cys Gly Gly Gly Ala Gln Ser Val Met Gly
    530                 535                 540

Ala Ser Gly Pro Pro Ala Gln Asp Val Leu Gly Ala Asp Leu Ala Ser
545                 550                 555                 560

Gln Leu Arg Ser Leu Gln Gly Lys Pro Ser Ala Phe Gly Asn Lys Pro
                565                 570                 575

Phe Gly Ser Ile Ser Pro
            580

<210>  7
<211>  451
<212>  PRT
<213>  Pseudozyma sp.

<400> 7

Met Arg Phe Phe Ser Arg Ala Ile Val Val Leu Ala Thr Ala Ala Thr
1               5                   10                  15

Val Ala Leu Ala Val Pro Met Gln Lys Arg Ala Gln Tyr Pro Asp Pro
            20                  25                  30

Asn Asp Asp Pro Phe Tyr Arg Val Pro Ala Asn Ile Asn Thr Tyr Ala
        35                  40                  45

Asn Gly Gln Val Ile Gln Ser Arg Ser Ala Thr Thr Asp Ile Gly Thr
    50                  55                  60

Gln Asn Asn Ala Ala Ser Phe Gln Leu Leu Tyr Arg Thr Thr Asp Thr
65                  70                  75                  80

Gln Asn Asn Ala Thr Ala Ser Val Ala Thr Val Trp Ile Pro Ser Lys
                85                  90                  95

Pro Ala Ser Pro Pro Lys Ile Phe Ser Tyr Gln Val Tyr Glu Asp Ala
            100                 105                 110

Thr Gln Leu Asp Cys Ala Pro Ser Tyr Asn Tyr Leu Ser Gly Phe Asp
        115                 120                 125

Gln Pro Gly Lys Gly Thr Val Ile Thr Asp Thr Pro Ile Ala Ile Ser
    130                 135                 140

Trp Ala Leu Gln Gln Gly Tyr Tyr Ala Val Ser Ser Asp His Glu Gly
145                 150                 155                 160

Phe Arg Ser Ala Phe Ile Ala Gly Tyr Glu Glu Gly Met Ala Ile Leu
                165                 170                 175

Asp Gly Val Arg Ala Leu Lys Asn Tyr Lys Ser Leu Pro Ser Asn Ser
            180                 185                 190

Ala Val Gly Phe Tyr Gly Tyr Ser Gly Gly Ala His Ala Thr Gly Trp
        195                 200                 205

Ala Val Ser Leu Ser Gly Ala Tyr Ala Ser Asp Leu Asn Ile Ile Gly
    210                 215                 220

Ala Ala Tyr Gly Gly Leu Pro Ala Ser Ala Lys Asp Thr Phe Leu Phe
225                 230                 235                 240

Leu Asn Ala Lys Ser Pro Phe Ala Gly Phe Ala Ile Ala Gly Val Ser
                245                 250                 255

Gly Leu Ala Leu Ala His Pro Asp Met Glu Ala Phe Ile Gln Pro Arg
            260                 265                 270

Leu Asn Ala Arg Gly Arg Gln Val Leu Gln Gln Ile Arg Ser Arg Gly
        275                 280                 285

Gln Cys Ile Gly Gln Val Ser Thr Gly Tyr Pro Phe Leu Asp Thr Phe
    290                 295                 300

Ser Leu Val Asn Asp Thr Asn Leu Leu Asn Glu Glu Pro Ile Val Ser
305                 310                 315                 320

Ile Leu Lys Ala Glu Thr Leu Val Gln Ser Glu Ala Gly Tyr Thr Val
                325                 330                 335

Pro Val Pro Lys Phe Pro Arg Phe Met Trp His Ala Leu Glu Asp Glu
            340                 345                 350

Ile Val Pro Phe Gln Pro Asp Ala Gln Tyr Val Lys Glu Gln Cys Ser
        355                 360                 365

Lys Gly Ala Asp Ile Asn Trp Asn Ile Tyr Pro Val Ala Glu His Ile
    370                 375                 380

Ser Ala Glu Leu Leu Gly Leu Val Pro Ala Leu Ala Trp Leu Glu Thr
385                 390                 395                 400

Ala Tyr Ala Gly Gln Ala Pro Arg Val Leu Cys Gly Val Gly Val Pro
                405                 410                 415

Val Pro Gln Pro Ser Ala Phe Thr Val Leu Gly Asp Lys Leu Ala Gln
            420                 425                 430

Gln Phe Lys Ser Leu Ala Gly Gln Gln Ser Ala Phe Gly Lys Thr Phe
        435                 440                 445

Pro Ser Ile
    450

<210>  8
<211>  459
<212>  PRT
<213>  Sporisorium reilianum

<400>  8

Met Arg Phe Phe Ala Gln Thr Leu Val Ala Leu Ala Ala Ala Ala Thr
1               5                   10                  15

Val Ser Leu Ala Ala Pro Leu Glu Arg Arg Ala Gln Phe Pro Asp Pro

            20                  25                  30

Asn Asp Asp Pro Phe Tyr Ser Ala Pro Ala Asn Ile Gly Ser Tyr Val
        35                  40                  45

Asn Gly Gln Val Ile Gln Ser Arg Ser Ala Thr Thr Asp Ile Gly Thr
    50                  55                  60

Ser Asn Asn Ala Ala Ser Phe Gln Leu Leu Tyr Arg Thr Thr Asn Thr
65                  70                  75                  80

Ser Asn Leu Pro Glu Ala Thr Val Ala Thr Val Trp Ile Pro Ala Lys
                85                  90                  95

Pro Ala Ser Pro Pro Lys Ile Phe Ser Tyr Gln Val Tyr Glu Asp Ala
            100                 105                 110

Thr Gln Leu Asn Cys Ala Pro Ser Tyr Ser Tyr Leu Ser Gly Leu Asp
        115                 120                 125

Glu Pro Gly Lys Gly Thr Val Ile Leu Asp Thr Pro Ile Val Ile Ser
    130                 135                 140

Trp Ala Leu Gln Gln Gly Tyr Tyr Val Val Ser Ala Asp His Glu Gly
145                 150                 155                 160

Pro Lys Ala Ala Phe Ile Ala Gly Cys Gln Glu Gly Arg Ala Ile Leu
                165                 170                 175

Asp Gly Val Arg Ala Leu Arg Asn Phe Gln Asn Leu Ala Ser Asn Ser
            180                 185                 190

Ala Val Gly Phe Tyr Gly Tyr Ser Gly Gly Gly His Ala Thr Gly Trp
        195                 200                 205

Ala Val Ser Leu Ala Gly Ser Tyr Ala Pro Asp Val Asn Ile Ile Gly
    210                 215                 220

Ala Ala Tyr Gly Gly Val Pro Thr Ser Thr Arg Asp Ile Phe Asn Phe
225                 230                 235                 240

Leu Asn Gly Gly Ala Phe Ala Gly Phe Ala Val Ala Gly Val Ser Gly
                245                 250                 255

Leu Gly Leu Ala Tyr Pro Glu Leu Glu Ala Tyr Ile Glu Pro Arg Leu
            260                 265                 270

Asn Ala Lys Gly Gln Asp Ala Leu Lys Arg Phe Arg Ser Arg Gly Tyr
        275                 280                 285

Cys Ile Gly Gln Val Val Thr Ser Glu Asn Phe Val Asp Ile Tyr Thr
    290                 295                 300

Leu Val Asn Asp Ser Asn Ile Leu Asn Glu Pro Ile Pro Ser Gln Val
305                 310                 315                 320

Leu Ala Lys Glu Thr Leu Leu Gln Thr Gln Ala Ser Tyr Thr Val Pro
                325                 330                 335

Val Pro Lys Ser Pro Arg Phe Ile Trp His Ala Leu Glu Asp Glu Ile
            340                 345                 350

Val Pro Phe Lys Pro Ala Gln Gln Tyr Val Thr Glu Gln Cys Ala Lys
        355                 360                 365

Gly Ala Asn Ile Asn Trp Asn Val Phe Pro Ile Ala Glu His Ile Ser
    370                 375                 380

Ala Glu Leu Phe Gly Leu Val Pro Gly Leu Asp Trp Leu Ser Lys Ala
385                 390                 395                 400

Tyr Arg Gly Gln Ala Pro Lys Val Ile Cys Gly Ser Ser Ile Pro Ala
                405                 410                 415

Ile Thr Gly Val Asn Ser Pro Ser Ala Gln Gln Val Leu Gly Ala Asp
            420                 425                 430

Leu Ala Gln Gln Leu Ser Asn Leu Asn Gly Lys Gln Ser Ala Phe Gly
        435                 440                 445

Lys Pro Tyr Gly Pro Ile Thr Pro Pro Thr Ala
    450                 455


<210>  9
<211>  458
<212>  PRT
<213>  Sporisorium reilianum

<400>  9

Met Arg Phe Ser Pro Gln Asn Trp Ile Ala Leu Ala Ile Ala Ala Thr
1               5                   10                  15

Ile Val Val Ala Ala Pro Asn Pro Gln Lys Asn Lys Arg Ala Pro Phe
            20                  25                  30

Pro Asp Pro Asn Asp Asp Asp Phe Tyr Lys Asn Pro Ala Asn Ile Thr
        35                  40                  45

Ser Tyr Ser Asn Gly Glu Ile Ile Arg Ser Arg Lys Ala Ala Thr Asp
50                  55                  60

Leu Gly Asn Leu Asn Asn Val Asp Ser Phe Gln Leu Ser Tyr Arg Thr
65                  70                  75                  80

Thr Asn Thr Gln Asn Glu Ala Gln Ala Asn Val Ala Thr Val Trp Leu
                85                  90                  95

Pro Ala Lys Pro Ala Ser Pro Pro Lys Ile Phe Phe Val Gln Leu Trp
            100                 105                 110

Glu Asp Ser Thr Gln Leu Asn Cys Ala Pro Ser Tyr Asn Tyr Leu Thr
        115                 120                 125

Gly Leu Asp Gln Pro Asn Lys Val Pro Ala Thr Ile Asp Thr Leu Val
    130                 135                 140

Ile Val Ser Trp Ala Leu Gln Gln Gly Tyr Tyr Val Val Ser Ser Asp
145                 150                 155                 160

His Ala Gly Pro Arg Ser Ala Phe Ile Gly Gly Trp Glu Asn Gly Met
                165                 170                 175

Ala Val Leu Asp Gly Val Arg Ala Leu Lys His Tyr Lys Ser Leu Pro
            180                 185                 190

Ser Asp Ser Ala Val Gly Phe Tyr Gly Tyr Ser Gly Gly Gly His Ala
        195                 200                 205

Thr Ala Arg Ala Val Asn Leu Gln Glu Ser Tyr Ala Pro Glu Leu Asn
    210                 215                 220

Val Val Ala Ala Ala Tyr Gly Gly Val Pro Thr Ser Ala Leu Leu Leu
225                 230                 235                 240

Phe Leu Asn Lys Gly Phe Val Ala Gly Phe Val Val Ala Gly Leu Ser
                245                 250                 255

Gly Ile Ser Leu Ala Tyr Pro Glu Val Glu Ala Tyr Leu Lys Pro Arg
            260                 265                 270

Leu Thr Ala Arg Gly Gln Gln Ala Leu Ala Gln Val Gln Ser Arg Asp
        275                 280                 285

Phe Cys Val Ala Asn Val Val Thr His Asn Asn Phe Val Asp Val Phe
    290                 295                 300

Ser Leu Val Asn Gln Asn Asp Phe Leu Asp Gln Asp Pro Val Arg Gln

305                 310                 315                 320

Val Ile Ala Arg Glu Thr Leu Leu Gln Ala Glu Ala Ser Tyr Thr Val
                325                 330                 335

Pro Val Pro Arg Phe Pro Arg Phe Met Trp His Gly Asn Ile Asp Glu
            340                 345                 350

Ile Val Pro Phe Ile Pro Asp Ala Asp Tyr Val Lys Glu Gln Cys Arg
        355                 360                 365

Lys Gly Ala Asn Ile Asn Trp Asn Val Leu Pro Leu Thr Gly His Ala
    370                 375                 380

Gly Ala Glu Ile Val Gly Leu Val Pro Ala Leu Asp Trp Met Gly Lys
385                 390                 395                 400

Val Phe Arg Gly Gln Thr Pro Lys Val Ser Cys Gly Asn Gly Val Pro
                405                 410                 415

Ala Ile Ser Gly Ile Asn Ser Pro Ser Ala Lys Gln Val Leu Gly Ser
            420                 425                 430

Asp Leu Ala Lys Arg Leu Glu Glu Leu Lys Ser Pro Gln Ser Ser Leu
        435                 440                 445

Val Lys Leu Phe Ser Gly Val Ala Pro Ser
    450                 455

<210>  10
<211>  444
<212>  PRT
<213>  Pseudozyma sp.

<400>  10

Met Gln Trp Ile Ser Leu Ala Ile Ala Ala Leu Ala Ala Thr Ala Thr
1               5                   10                  15

Ile Ala Ile Ala Ala Gly Met Glu Pro Arg Thr Leu Pro His Pro Thr
            20                  25                  30

Glu Asp Pro Phe Tyr Asn Val Pro Ser Asn Val Asp Ser Tyr Asp Asn
        35                  40                  45

Gly Gln Val Ile Met Ser Arg Arg Val Ser Thr Ser Ile Gly Thr Met
    50                  55                  60

Glu Asn Val Ala Ser His Gln Val Leu Tyr Arg Thr Thr Asn Thr Gln
65                  70                  75                  80

Asn His Ala Ser Ala Thr Val Ala Thr Ile Trp Thr Pro Ser Lys Pro
                85                  90                  95

Val Ser Pro Pro Lys Ile Leu Ser Tyr Gln Leu Tyr Met Asp Ala Thr
            100                 105                 110

Gln Leu Asn Cys Ala Pro Ser Tyr Ala Phe Leu Ser Gly Ile His Asn
        115                 120                 125

Thr Glu Lys Ala Thr Thr Ile Leu Asp Thr Pro Ile Val Ile Ser Trp
    130                 135                 140

Ala Leu Lys His Gly Phe Tyr Val Val Ser Pro Asp His Glu Gly Pro
145                 150                 155                 160

Arg Ser Ala Phe Ile Ala Gly Tyr Glu Glu Gly Arg Ala Ile Leu Asp
                165                 170                 175

Gly Val Arg Ala Leu Lys Asn Phe Lys Glu Leu Pro Glu Asn Ser Ala
            180                 185                 190

Val Gly Phe Tyr Gly Tyr Ser Gly Gly Gly His Ala Thr Ala Trp Ala
        195                 200                 205

Ala Ser Leu Ser Gly Gly Tyr Ala Pro Glu Leu Asn Ile Ile Gly Ala
    210                 215                 220

Ala Tyr Gly Gly Val Pro Thr Ser Thr Arg Met Ile Ala Asp Phe Leu
225                 230                 235                 240

Asn Gly Lys Ser Val Phe Ser Gly Phe Ala Leu Ala Gly Ile Ser Gly
                245                 250                 255

Leu Ala Arg Ala Tyr Pro Glu Met Glu Ala Phe Ile Leu Pro Arg Leu
            260                 265                 270

Asn Ala Glu Gly Gln Lys Thr Phe Glu Arg Ile Gly Ser Arg Gly Glu
        275                 280                 285

Cys Ile Ala Gln Val Ala Phe Gly Tyr Pro His Leu Asp Phe Tyr Thr
    290                 295                 300

Leu Val Asn Asp Thr Asn Met Met Ser Glu Glu Pro Ile Lys Ser Ile
305                 310                 315                 320

Leu Ser Val Glu Thr Leu Leu Lys Ser Glu Ala Ser Tyr Thr Val Pro
                325                 330                 335

Val Arg Lys Trp Pro Arg Tyr Ile Trp His Ala Leu Glu Asp Glu Ile
            340                 345                 350

Val Pro Phe Ala Pro Ala Ser Gln Tyr Val Lys Glu Gln Cys Ala Glu
        355                 360                 365

Gly Ala Asp Ile Asn Trp Asn Val Leu Pro Val Gln Glu His Val Ser
    370                 375                 380

Ala Glu Val Thr Gly Ile Ala Pro Ala Ile Ala Trp Leu Ser Glu Ala
385                 390                 395                 400

Phe Gln Gly Lys Ala Pro Lys Val Pro Cys Gly Ala Gly Asn Leu Pro
                405                 410                 415

Val Ala Pro Ser Val Lys Glu Val Leu Gly Asp Asp Leu Val Asn His
            420                 425                 430

Leu His Asn Leu Ala Gly Gln Val Lys Ala His Leu
        435                 440

<210>  11
<211>  465
<212>  PRT
<213>  Candida parapsilosis

<400>  11

Met Arg Tyr Phe Ala Ile Ala Phe Leu Leu Ile Asn Thr Ile Ser Ala
1               5                   10                  15

Phe Val Leu Ala Pro Lys Lys Pro Ser Gln Asp Asp Phe Tyr Thr Pro
            20                  25                  30

Pro Gln Gly Tyr Glu Ala Gln Pro Leu Gly Ser Ile Leu Lys Thr Arg
        35                  40                  45

Asn Val Pro Asn Pro Leu Thr Asn Val Phe Thr Pro Val Lys Val Gln
    50                  55                  60

Asn Ala Trp Gln Leu Leu Val Arg Ser Glu Asp Thr Phe Gly Asn Pro
65                  70                  75                  80

Asn Ala Ile Val Thr Thr Ile Ile Gln Pro Phe Asn Ala Lys Lys Asp
                85                  90                  95

Lys Leu Val Ser Tyr Gln Thr Phe Glu Asp Ser Gly Lys Leu Asp Cys
            100                 105                 110

Ala Pro Ser Tyr Ala Ile Gln Tyr Gly Ser Asp Ile Ser Thr Leu Thr
        115                 120                 125

Thr Gln Gly Glu Met Tyr Tyr Ile Ser Ala Leu Leu Asp Gln Gly Tyr
    130                 135                 140

Tyr Val Val Thr Pro Asp Tyr Glu Gly Pro Lys Ser Thr Phe Thr Val
145                 150                 155                 160

Gly Leu Gln Ser Gly Arg Ala Thr Leu Asn Ser Leu Arg Ala Thr Leu
                165                 170                 175

Lys Ser Gly Asn Leu Thr Gly Val Ser Ser Asp Ala Glu Thr Leu Leu
            180                 185                 190

Trp Gly Tyr Ser Gly Gly Ser Leu Ala Ser Gly Trp Ala Ala Ala Ile
        195                 200                 205

Gln Lys Glu Tyr Ala Pro Glu Leu Ser Lys Asn Leu Leu Gly Ala Ala
    210                 215                 220

Leu Gly Gly Phe Val Thr Asn Ile Thr Ala Thr Ala Glu Ala Val Asp
225                 230                 235                 240

Ser Gly Pro Phe Ala Gly Ile Ile Ser Asn Ala Leu Ala Gly Ile Gly
                245                 250                 255

Asn Glu Tyr Pro Asp Phe Lys Asn Tyr Leu Leu Lys Lys Val Ser Pro
            260                 265                 270

Leu Leu Ser Ile Thr Tyr Arg Leu Gly Asn Thr His Cys Leu Leu Asp
        275                 280                 285

Gly Gly Ile Ala Tyr Phe Gly Lys Ser Phe Phe Ser Arg Ile Ile Arg
    290                 295                 300

Tyr Phe Pro Asp Gly Trp Asp Leu Val Asn Gln Glu Pro Ile Lys Thr
305                 310                 315                 320

Ile Leu Gln Asp Asn Gly Leu Val Tyr Gln Pro Lys Asp Leu Thr Pro
                325                 330                 335

Gln Ile Pro Leu Phe Ile Tyr His Gly Thr Leu Asp Ala Ile Val Pro
            340                 345                 350

Ile Val Asn Ser Arg Lys Thr Phe Gln Gln Trp Cys Asp Trp Gly Leu
        355                 360                 365

Lys Ser Gly Glu Tyr Asn Glu Asp Leu Thr Asn Gly His Ile Thr Glu
    370                 375                 380

Ser Ile Val Gly Ala Pro Ala Ala Leu Thr Trp Ile Ile Asn Arg Phe
385                 390                 395                 400

Asn Gly Gln Pro Pro Val Asp Gly Cys Gln His Asn Val Arg Ala Ser
                405                 410                 415

Asn Leu Glu Tyr Pro Gly Thr Pro Gln Ser Ile Lys Asn Tyr Phe Glu
            420                 425                 430

Ala Ala Leu His Ala Ile Leu Gly Phe Asp Leu Gly Pro Asp Val Lys
        435                 440                 445

Arg Asp Lys Val Thr Leu Gly Gly Leu Leu Lys Leu Glu Arg Phe Ala
    450                 455                 460

Phe
465

<210>  12
<211>  543
<212>  PRT
<213>  Acinetobacter calcoaceticus

<400>  12

Met Ser Gln Lys Met Asp Phe Asp Ala Ile Val Ile Gly Gly Gly Phe
1               5                   10                  15

Gly Gly Leu Tyr Ala Val Lys Lys Leu Arg Asp Glu Leu Glu Leu Lys
            20                  25                  30

Val Gln Ala Phe Asp Lys Ala Thr Asp Val Ala Gly Thr Trp Tyr Trp
        35                  40                  45

Asn Arg Tyr Pro Gly Ala Leu Thr Asp Thr Glu Thr His Leu Tyr Cys
    50                  55                  60

Tyr Ser Trp Asp Lys Glu Leu Leu Gln Ser Leu Glu Ile Lys Lys Lys
65                  70                  75                  80

Tyr Val Gln Gly Pro Asp Val Arg Lys Tyr Leu Gln Gln Val Ala Glu
                85                  90                  95

Lys His Asp Leu Lys Lys Ser Tyr Gln Phe Asn Thr Ala Val Gln Ser
            100                 105                 110

Ala His Tyr Asn Glu Ala Asp Ala Leu Trp Glu Val Thr Thr Glu Tyr
        115                 120                 125

Gly Asp Lys Tyr Thr Ala Arg Phe Leu Ile Thr Ala Leu Gly Leu Leu

130                 135                 140

Ser Ala Pro Asn Leu Pro Asn Ile Lys Gly Ile Asn Gln Phe Lys Gly
145                 150                 155                 160

Glu Leu His His Thr Ser Arg Trp Pro Asp Asp Val Ser Phe Glu Gly
                165                 170                 175

Lys Arg Val Gly Val Ile Gly Thr Gly Ser Thr Gly Val Gln Val Ile
            180                 185                 190

Thr Ala Val Ala Pro Leu Ala Lys His Leu Thr Val Phe Gln Arg Ser
        195                 200                 205

Ala Gln Tyr Ser Val Pro Ile Gly Asn Asp Pro Leu Ser Glu Glu Asp
    210                 215                 220

Val Lys Lys Ile Lys Asp Asn Tyr Asp Lys Ile Trp Asp Gly Val Trp
225                 230                 235                 240

Asn Ser Ala Leu Ala Phe Gly Leu Asn Glu Ser Thr Val Pro Ala Met
                245                 250                 255

Ser Val Ser Ala Glu Glu Arg Lys Ala Val Phe Glu Lys Ala Trp Gln
            260                 265                 270

Thr Gly Gly Gly Phe Arg Phe Met Phe Glu Thr Phe Gly Asp Ile Ala
        275                 280                 285

Thr Asn Met Glu Ala Asn Ile Glu Ala Gln Asn Phe Ile Lys Gly Lys
    290                 295                 300

Ile Ala Glu Ile Val Lys Asp Pro Ala Ile Ala Gln Lys Leu Met Pro
305                 310                 315                 320

Gln Asp Leu Tyr Ala Lys Arg Pro Leu Cys Asp Ser Gly Tyr Tyr Asn
                325                 330                 335

Thr Phe Asn Arg Asp Asn Val Arg Leu Glu Asp Val Lys Ala Asn Pro
            340                 345                 350

Ile Val Glu Ile Thr Glu Asn Gly Val Lys Leu Glu Asn Gly Asp Phe
        355                 360                 365

Val Glu Leu Asp Met Leu Ile Cys Ala Thr Gly Phe Asp Ala Val Asp
    370                 375                 380

Gly Asn Tyr Val Arg Met Asp Ile Gln Gly Lys Asn Gly Leu Ala Met
385                 390                 395                 400

Lys Asp Tyr Trp Lys Glu Gly Pro Ser Ser Tyr Met Gly Val Thr Val
                405                 410                 415

Asn Asn Tyr Pro Asn Met Phe Met Val Leu Gly Pro Asn Gly Pro Phe
            420                 425                 430

Thr Asn Leu Pro Pro Ser Ile Glu Ser Gln Val Glu Trp Ile Ser Asp
        435                 440                 445

Thr Ile Gln Tyr Thr Val Glu Asn Asn Val Glu Ser Ile Glu Ala Thr
    450                 455                 460

Lys Glu Ala Glu Glu Gln Trp Thr Gln Thr Cys Ala Asn Ile Ala Glu
465                 470                 475                 480

Met Thr Leu Phe Pro Lys Ala Gln Ser Trp Ile Phe Gly Ala Asn Ile
                485                 490                 495

Pro Gly Lys Lys Asn Thr Val Tyr Phe Tyr Leu Gly Gly Leu Lys Glu
            500                 505                 510

Tyr Arg Ser Ala Leu Ala Asn Cys Lys Asn His Ala Tyr Glu Gly Phe
        515                 520                 525

Asp Ile Gln Leu Gln Arg Ser Asp Ile Lys Gln Pro Ala Asn Ala
    530                 535                 540

<210> 13
<211> 249
<212> PRT
<213> Lactobacillus kefir

<400> 13

Met Thr Asp Arg Leu Lys Gly Lys Val Ala Ile Val Thr Gly Gly Thr
1               5                   10                  15

Leu Gly Ile Gly Leu Ala Ile Ala Asp Lys Phe Val Glu Glu Gly Ala
            20                  25                  30

Lys Val Val Ile Thr Gly Arg His Ala Asp Val Gly Glu Lys Ala Ala
        35                  40                  45

Lys Ser Ile Gly Gly Thr Asp Val Ile Arg Phe Val Gln His Asp Ala
    50                  55                  60

Ser Asp Glu Ala Trp Thr Lys Leu Phe Asp Thr Thr Glu Glu Ala Phe
65                  70                  75                  80

Gly Pro Val Thr Thr Val Val Asn Asn Ala Gly Ile Ala Val Ser Lys
                85                  90                  95

Ser Val Glu Asp Thr Thr Thr Glu Glu Trp Arg Lys Leu Leu Ser Val
            100                 105                 110

Asn Leu Asp Gly Val Phe Phe Gly Thr Arg Leu Gly Ile Gln Arg Met
        115                 120                 125

Lys Asn Lys Gly Leu Gly Ala Ser Ile Ile Asn Ser Ser Ile Glu Gly
    130                 135                 140

Phe Val Gly Asp Pro Thr Leu Gly Ala Tyr Asn Ala Ser Lys Gly Ala
145                 150                 155                 160

Val Arg Ile Met Ser Lys Ser Ala Ala Leu Asp Cys Ala Leu Lys Asp
                165                 170                 175

Tyr Asp Val Arg Val Asn Thr Val His Pro Gly Tyr Ile Lys Thr Pro
            180                 185                 190

Leu Val Asp Asp Leu Glu Gly Ala Glu Glu Met Met Ser Gln Arg Thr
        195                 200                 205

Lys Thr Pro Met Gly His Ile Gly Glu Pro Asn Asp Ile Ala Trp Ile
    210                 215                 220

Cys Val Tyr Leu Ala Ser Asp Glu Ser Lys Phe Ala Thr Ala Glu Phe
225                 230                 235                 240

Val Val Asp Gly Gly Tyr Thr Ala Gln
                245